# EUROPÄISCHE PATENTSCHRIFT

(11) **EP 1 611 094 B1**
(45) Veröffentlichungstag und Bekanntmachung des Hinweises auf die Patenterteilung: **20.06.2007**
(21) Anmeldenummer: 04718288.6
(22) Anmeldetag: 08.03.2004
(51) Int. Cl.: C07D 401/04, C07D 231/10, A61K 31/435

(54) **PYRAZOLVERBINDUNGEN**
PYRAZOLE COMPOUNDS
COMPOSES PYRAZOLE

(30) Priorität: 05.04.2003 DE 10315571
(43) Veröffentlichungstag der Anmeldung: 04.01.2006
(73) Patentinhaber: Merck Patent GmbH, 64293 Darmstadt (DE)
(72) Erfinder: SCHADT, Oliver, 63517 Rodenbach (DE); SCHIEMANN, Kai, 64342 Seehein-Jugenheim (DE); VAN AMSTERDAM, Christoph, 64295 Darmstadt (DE); BARTOSZYK, Gerd, 64331 Weiterstadt (DE); SEYFRIED, Christoph, 64342 Seeheim-Jugenheim (DE)
(86) Internationale Anmeldenummer: PCT/EP2004/002352
(87) Internationale Veröffentlichungsnummer: WO 2004/089888

(56) Entgegenhaltungen:
- WO-A-01/32626
- WO-A-03/088958
- DATABASE BEILSTEIN [Online] BEILSTEIN INSTITUTE FOR ORGANIC CHEMISTRY, FRANKFURT-MAIN, DE; 27. Juni 1988 (1988-06-27), VEIBEL: XP002283830 gefunden im CROSSFIRE Database accession no. BRN 268510; 232330

## Beschreibung

Die Erfindung betrifft Verbindungen der Formel 1 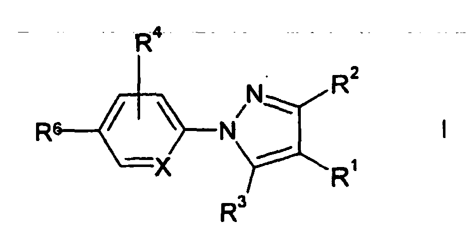 worin
- R², R⁴: H, A, Hal, Cycloalkyl mit 3 bis 7 C-Atomen, CF₃, N0₂, CN, OCF₃, OA, NHA, NA₂, NH₂,
- R³, R⁶: (CH₂)ₙHet, (CH₂)ₙAr,
- R¹: H oder ein organischer Rest, insbesondere (CH₂)ₙCO₂R⁵, (CH₂)ₙCOHet, CHO, (CH₂)ₙOR⁵, (CH₂)ₙHet, (CH₂)ₙN(R⁵)₂, CH=N-OA, CH₂CH=N-OA, (CH₂)ₙNHOA, (CH₂)ₙ(R⁵)Het, (CH₂)ₙCH=N-Het, (CH₂)ₙOCOR⁵, (CH₂)ₙN(R⁵)CH₂CH₂OR⁵, (CH₂)ₙN(R⁵)CH₂CH₂OCF₃, (CH₂)ₙN(R⁵)C(R⁵)COOR⁵, (CH₂)ₙN(R⁵)CH₂COHet, (CH₂)ₙN(R⁵)CH₂Het, (CH₂)ₙN(R⁵)CH₂CH₂Het, (CH₂)ₙN(R⁵)CH₂CH₂N(R⁵)CH₂COOR⁵, (CH₂)ₙN(R⁵)CH₂CH₂N(R⁵)₂, CH=CHCOOR⁵, CH=CHCH₂NR⁵Het, CH=CHCH₂N(R⁵)₂, CH=CHCH₂OR⁵ oder (CH₂)ₙN(R⁵)Ar,
- R⁵: H oder A
- A: geradkettiges oder verzweigtes Alkyl oder Alkoxy mit 1 bis 10 C-Atomen, Alkenyl oder Alkenyloxyalkyl mit 2 bis 10 C-Atomen,
- Het: einen organischen Rest, insbesondere einen unsubstituierten oder einfach oder mehrfach durch A und/oder Hal substituierten, gesättigten, ungesättigten oder aromatischen mono- oder bicyclischen heterocyclischen oder linearen oder verzweigten ein oder mehrere Heteroatome enthaltenden organischen Rest,
- Ar: einen organischen aromatischen Rest, insbesondere einen unsubstituierten oder einfach oder mehrfach durch A und/oder Hal, OR⁵, OOCR⁵, COOR⁵, CON(R⁵)₂, CN, NO₂, NH₂, NHCOR⁵, CF₃, SO₂CH₃ oder einer ringbildenden Gruppe -OCH₂O-, -O(CH₂)₂O- oder -OC(CH₃)₂O- substituierten Phenylrest,
- n: 0, 1, 2, 3, 4 oder 5
und
- Hal: F, Cl, Br oder I
bedeuten
wobei im Fall das X die Bedeutung CH aufweist, R² und R⁴ nicht gleichzeitig H bedeuten,
sowie deren Salze und Solvate, Enantiomere, Racemate und andere Mischungen der Enantiomere, insbesondere deren physiologisch verträglichen Salze und Solvate.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze und Solvate bei guter Verträglichkeit sehr wertvolle pharmakologische Eigenschaften besitzen.

Gegenstand der Erfindung sind insbesondere die in den Beispielen genannten Verbindungen, die die in der vorliegenden Anmeldung geschilderten Eigenschaften und Verwendungsmöglichkeiten der Verbindungen der Formel 1 aufweisen.

Insbesondere eignen sich die erfindungsgemäßen Verbindungen der Formel I als Liganden von 5 HT-Rezeptoren, so daß die erfindungsgemäßen Verbindungen, sowie deren Salze und Solvate, Enantiomere und Racemate, insbesondere deren physiologisch verträglichen Salze und Solvate, zur Behandlung und Prophylaxe von Krankheiten geeignet sind, die durch die Bindung der Verbindungen der Formel I an 5 HT-Rezeptoren beeinflusst werden können.

Ähnliche Verbindungen sind beispielsweise aus DE 2201889, DE 2258033 oder DE 2906252 sowie WO 01/32626 bekannt.

Insbesondere eignen sich die erfindungsgemäßen Verbindungen der Formel I als Liganden von 5 HT2A- und/oder 5HT2C-Rezeptoren und können in der Human- und Veterinärmedizin zur Prophylaxe und Behandlung verschiedener Krankheiten des zentralen Nervensystems, wie z.B. Schizophrenie, Depression, Demenz, Dyskinesie, Parkinsonschen Krankheit, Morbus Alzheimer, Lewy Bodies Dementia, Huntington, Tourette Syndrom, Angst, Lern- und Erinnerungseinschränkungen, neurodegenerativen Erkrankungen und anderen kognitiven Beeinträchtigungen, sowie Nikotinabhängigkeit und Schmerzen verwendet werden.

Insbesondere bevorzugt werden die Verbindungen der Formel I und/oder ihre physiologisch unbedenklichen Salze oder Solvate zur Herstellung eines Arzneimittels zur Prophylaxe und/oder Behandlung von Psychosen, neurologischen Störungen, amyotropher Lateralsklerose, Essstörungen wie Bulimie, nervöser Anorexie, des prämenstrualen Syndroms und/oder zur positiven Beeinflussung von Zwangsverhalten (obsessive-compulsive disorder, OCD) verwendet.

Es wurde gefunden, dass die Verbindungen der Formel 1 und ihre physiologisch unbedenklichen Salze und Solvate bei guter Verträglichkeit wertvolle pharmakologische Eigenschaften besitzen, da sie Wirkungen auf das Zentralnervensystem besitzen. Die Verbindungen weisen eine starke Affinität zu 5-HT_{2A}-Rezeptoren aufweisen, weiterhin zeigen sie 5-HT_{2A}-Rezeptor-antagonistische Eigenschaften.

Bevorzugt ist daher Verwendung der Verbindungen der Formel I und/oder von deren physiologisch unbedenklichen Salzen und Solvaten zur Herstellung eines Arzneimittels mit 5-HT-Rezeptorantagonistischer, insbesondere 5-HT_{2A}-Rezeptorantagonistischer Wirkung.

Zum in-vitro Nachweis der Affinität zu 5-HT_{2A}-Rezeptoren kann beispielsweise folgender Test (Beispiel A1) herangezogen werden. Die 5-HT_{2A} Rezeptoren werden sowohl [³H]Ketanserin (eine Substanz, bekannt für ihre Affinität zum Rezeptor) als auch der Testverbindung ausgesetzt. Die Abnahme der Affinität von [³H]Ketanserin zum Rezeptor ist ein Anzeichen für die Affinität der Testsubstanz zum 5-HT_{2A} Rezeptor. Der Nachweis erfolgt analog der Beschreibung von J.E. Leysen et al., Molecular Pharmacology, 1982, 21: 301-314 oder wie z.B. auch in EP 0320983 beschrieben.

Die Wirksamkeit der erfindungsgemäßen Verbindungen als 5-HT_{2A} Rezeptor-Antagonisten kann in vitro analog W. Feniuk et al., Mechanisms of 5-hydroxytryptamine-induced vasoconstriction, in: The Peripheral Actions of 5-Hydroxytryptamine, ed. Fozard JR, Oxford University Press, New York, 1989, p.110, gemessen werden. So wird die Kontraktilität der Rattenschwanzarterie, hervorgerufen durch 5-Hydroxytryptamin, durch 5-HT_{2A} Rezeptoren vermittelt. Für das Testsystem werden Gefäßringe, präpariert aus der ventralen Rattenschwanzarterie, in einem Organbad mit einer sauerstoffgesättigteri Lösung einer Perfusion unterzogen. Durch Eintrag ansteigender Konzentrationen an 5-Hydroxytryptamin in die Lösung erhält man eine Antwort auf die kumulative Konzentration an 5-HT.. Danach wird die Testverbindung in geeigneten Konzentrationen in das Organbad gegeben und eine zweite Konzentrationskurve für 5-HT gemessen. Die Stärke der Testverbindung auf die Verschiebung der 5-HT induzierten Konzentrationskurve zu höheren 5-HT Konzentrationen ist ein Maß für die 5-HT_{2A}-Rezeptor-anatgonistische Eigenschaft in vitro.

Die 5-HT_{2A}-antagonistische Eigenschaft kann in vivo analog M.D.Serdar et al., Psychopharmacology, 1996, 128: 198-205, bestimmt werden.

Die Verbindungen der Formel I eignen sich daher sowohl in der Veterinärals auch in der Humanmedizin zur Behandlung von Funktionsstörungen des Zentralnervensystems sowie von Entzündungen. Sie können zur Prophylaxe und zur Bekämpfung der Folgen cerebraler Infarktgeschehen (apoplexia cerebri) wie Schlaganfall und cerebraler Ischämien sowie zur Behandlung extrapyramidal-motorischer Nebenwirkungen von Neuroleptika sowie des Morbus Parkinson, zur akuten und symptomatischen Therapie der Alzheimer Krankheit sowie zur Behandlung der amyotrophen Lateralsklerose verwendet werden. Ebenso eignen sie sich als Therapeutika zur Behandlung von Hirn- und Rückenmarkstraumata. Insbesondere sind sie jedoch geeignet als Arzneimittelwirkstoffe für Anxiolytika, Antidepressiva, Antipsychotika, Neuroleptika, Antihypertonika und/oder zur positiven Beeinflussung von Zwangsverhalten (obsessive-compulsive disorder, OCD; z.B. WO 9524194), Angstzuständen sowie physiologischen Veränderungen, die mit Angstzuständen einhergehen wie z.B. Tachycardie, Tremor oder Schwitzen (z.B. EP 319962), Panikattacken, Psychosen, Schizophrenie, Anorexie, zwanghaften Wahnvorstellungen, Agoraphobie, Migräne, der Alzheimer Krankheit, Schlafstörungen wie auch Schlafapnoe, tardiver Dyskinesien, Lernstörungen, altersabhängiger Erinnerungsstörungen, Essstörungen wie Bulimie, Drogenmissbrauch wie z.B. von Alkohol, Opiaten, Nikotin, Psychostimulantien wie z.B. Kokain oder Amphetaminen (z.B. US 6004980), Sexualfunktionsstörungen, Schmerzuständen aller Art und Fibromyalgie (z.B. WO 9946245).
Die Verbindungen der Formel I eignen sich zur Behandlung extrapyramidaler Nebenwirkungen (extrapyramidal side effects EPS) bei der neuroleptischen Drogentherapie. EPS ist gekennzeichnet durch Parkinson-ähnliche Syndrome, Akathisie und dystonische Reaktionen (z.B. EP 337136). Weiter sind sie geeignet zur Behandlung der nervösen Anorexie, Angina, Reynaud's Phänomen, koronaren Vasospasmen, bei der Prophylaxe von Migräne (z.B. EP 208235), Schmerz und Neuralgien (z.B. EP 320983), zur Behandlung des Rett-Syndroms mit autistischen Charakterzügen, des Asperger-Syndroms, des Autismus und autistischen Störungen, bei Konzentrationsmangelzuständen, Entwicklungsstörungen, Hyperaktivitätszuständen mit mentaler Unterentwicklung und stereotypen Verhaltenszuständen (z.B. WO 9524194).

Desweiteren sind sie geeignet zur Behandlung von endokrinen Erkrankungen wie Hyperprolactinaemie, ferner bei Vasospasmen, thrombotischen Erkrankungen (z.B. WO 9946245), Hypertension und gastrointestinalen Erkrankungen.
Ferner sind sie geeignet zur Behandlung cardiovaskulärer Erkrankungen sowie extrapyramidaler Symptome wie in der WO 99/11641 auf Seite 2, Zeile 24-30 beschrieben.
Die erfindungsgemäßen Verbindungen eignen sich weiter zur Verminderung des Augeninnendruckes und zur Glaucombehandlung.
Sie sind auch zur Prophylaxe und Behandlung von Vergiftungserscheinungen bei der Gabe von Ergovalin bei Tieren geeignet.
Die Verbindungen eignen sich weiterhin zur Behandlung von Erkrankungen des Herz-Kreislaufsystems (WO 99/11641, Seite 3, Zeile 14-15). Die erfindungsgemäßen Verbindungen können auch zusammen mit anderen Wirkstoffen in der Behandlung der Schizophrenie eingesetzt werden. Als andere Wirkstoffe kommen die in der WO 99/11641 auf Seite 13, Zeile 20-26 genannten Verbindungen in Frage.

Andere Verbindungen, die ebenfalls 5-HT₂-antagonistische Wirkungen zeigen, sind beispielweise in der EP 0320983 beschrieben.
In der WO 99/11641 sind Phenylindolderivate mit 5-HT₂-antagonistischen Eigenschaften beschrieben.

Keines der oben genannten Dokumente beschreibt jedoch die erfindungsgemäßen Verbindungen der Formel I oder deren Verwendung als Liganden von 5 HT-Rezeptoren.

Die Verbindungen der Formel I können als Arzneimittelwirkstoffe in der Human- und Veterinärmedizin eingesetzt werden. Ferner können sie als Zwischenprodukte zur Herstellung weiterer Arzneimittelwirkstoffe eingesetzt werden.

Gegenstand der Erfindung sind dementsprechend die Verbindungen der Formel I sowie deren Verwendung in der Human- und Tiermedizin.

Ein weiterer Gegenstand der vorliegenden Erfindung ist ein Verfahren zur Herstellung von Verbindungen der Formel IA 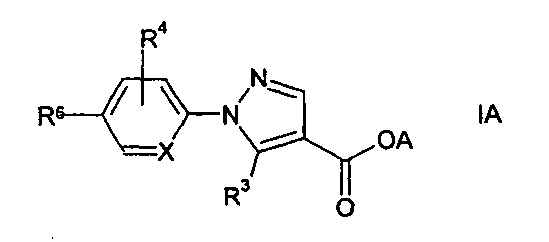 sowie deren Salze und Solvate, das dadurch gekennzeichnet ist, daß man eine Verbindung der Formel II 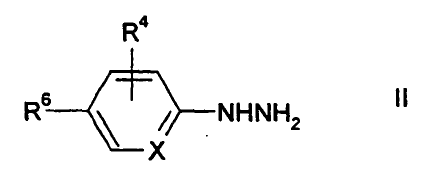 oder deren Säureadditionssalze
worin
R⁴, R⁶ und X die oben angegebenen Bedeutungen haben,
mit einer Verbindung der Formel III 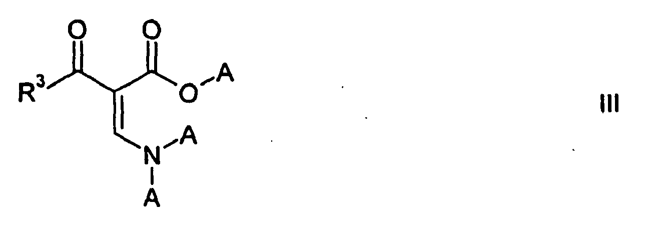 worin
A und R³ die oben angegebenen Bedeutungen haben,
umsetzt und/oder daß man eine basische Verbindung der Formel IA durch Behandeln mit einer Säure in eines ihrer Salze überführt.

Ein weiter Gegenstand der vorlegenden Erfindung ist ein Verfahren zur Herstellung von Verbindungen der Formel IB 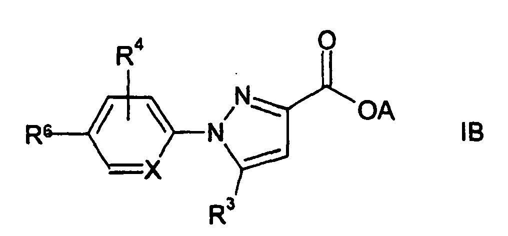 sowie deren Salze und Solvate, das dadurch gekennzeichnet ist, daß man eine Verbindung der Formel II 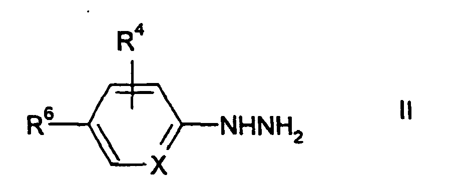 oder deren Säureadditionssalze
worin
R⁴, R⁶ und X die oben angegebenen Bedeutungen haben,
mit einer Verbindung der Formel IV 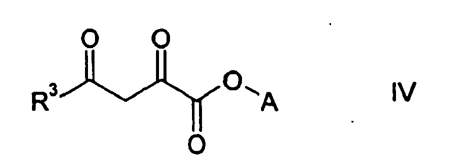 worin
A und R³ die oben angegebenen Bedeutungen haben, umsetzt
und/oder daß man eine basische Verbindung der Formel IB durch Behandeln mit einer Säure in eines ihrer Salze überführt.

Die Verbindungen der Formeln IA und IB können durch übliche Methoden in die weiteren Verbindungen der Formel I überführt werden. Insbesondere können die Verbindungen der Formel IA und IB durch Anwendung von Reduktionsmitteln wie z.B. Lithiumaluminiumhydrid in die entsprechenden Alkohole der Formeln IC und ID 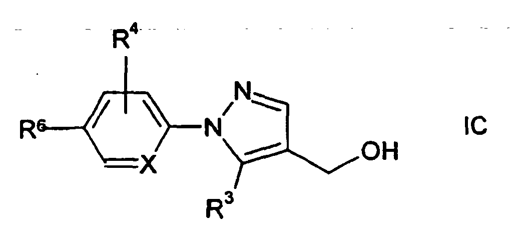 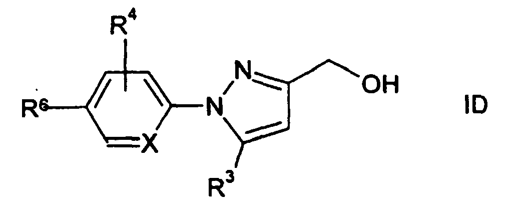 überführt werden, die z.B. mit MnO₂ zu den Verbindungen IE und IF oxidiert werden können.

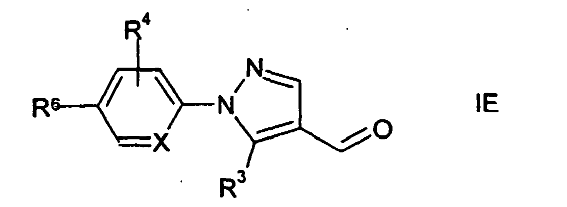

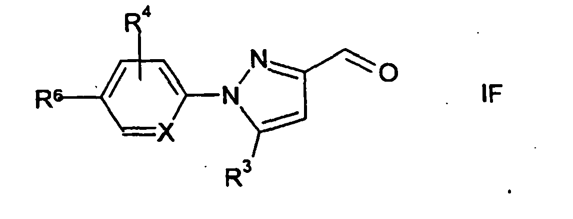

Die Verbindungen der Formeln IE und IF können ihrerseits nach bekannten Verfahren mit entsprechenden Nucleophilen wie z.B. Stickstoffbasen, insbesondere Hydroxylamin, 0-Methylhydroxylamin, Morpholin, Piperidin, Piperazin, N-Methylpiperazin, 4-Methylpiperazin-1-ylamin, Pyrrolidin, Pyrazolidin oder Imidazolidin, gegebenenfalls in Gegenwart eines Reduktionsmittels wie Natriumtriacetoxyborhydrid aminiert oder zu den entsprechenden lminen umgesetzt werden. Weiterhin können die Verbindungen der Formeln IE und IF durch Wittig-Reaktion mit Methoxymethyltriphenylphosphoniumsalzen zu den entsprechenden Enolethern umgesetzt werden; die durch Behandlung mit einer Säure in die homologisierten Aldehyde IG und IH 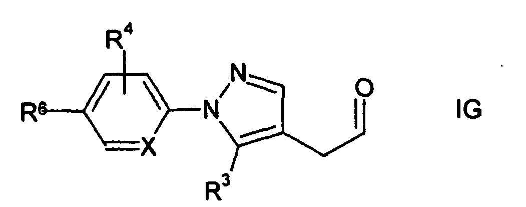 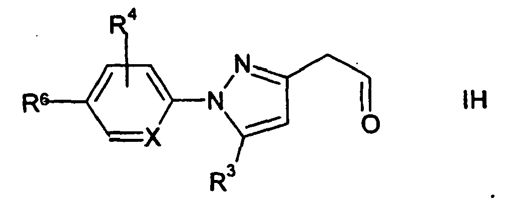 überführt werden können. Die Verbindungen der Formel IG und IH können analog zu den Verbindungen der Fomeln IE und IF zu den weiteren Verbindungen der Formel I umgesetzt werden.

Die neuen Verbindungen der Formel II, lll, IV und V sind ebenfalls Gegenstand der Erfindung.

Unter Solvaten der Verbindungen der Formel I werden Anlagerungen von inerten Lösungsmittelmolekülen an die Verbindungen der Formel I verstanden, die sich aufgrund ihrer gegenseitigen Anziehungskraft ausbilden. Solvate sind z.B. Mono- oder Dihydrate oder Alkoholate.

Vor- und nachstehend haben die Reste X, A, Ar, Het, n, R¹, R², R³, R⁴, R⁵ und R⁶ die bei der Formel I angegebenen Bedeutungen, sofern nicht ausdrücklich etwas anderes angegeben ist.

X bedeutet vorzugsweise N.

R⁶ steht bevorzugt für (CH₂)ₙAr, insbesondere für Ar. Ganz besonders bevorzugt bedeutet R⁵ Phenyl, 2-, 3- oder 4-Cyanophenyl, 2-, 3- oder 4-Fluorphenyl, 2-, 3- oder 4-Methyl-, Ethyl-, n-Propyl- oder n-Butylphenyl, 2,3-, 2,4-, 2,5-, 2,6-, 3,4-, 3,5-oder 3,6-Difluor- , Dichlor- oder Dicyanophenyl,3,4,5-Trifluorphenyl, 3,4,5-Trimethoxy-oder Triethoxyphenyl, Thiophen-2-yl oder Thiophen-3-yl.

R³ bedeutet vorzugsweise (CH₂)ₙHet oder (CH₂)ₙAr, insbesondere (CH₂)ₙAr. Ganz besonders bevorzugt bedeutet R³ Phenyl, 2-, 3- oder 4-Cyanophenyl, 2-, 3- oder 4-Fluorphenyl, 2-, 3- oder 4-Methyl-, Ethyl-, n-Propyl- oder n-Butylphenyl, 2,3-, 2,4-, 2,5-, 2,6-Difluor- oder Dicyanophenyl, Thiophen-2-yl oder Thiophen-3-yl, 2-, 3- oder 4-Pyridyl, 2-, 4- oder 5-Oxazolyl, 2-, 4- oder 5-Thiazolyl, Chinolinyl, Isochinolinyl, 2- oder 4-Pyridazyl, 2-, 4- oder 5-Pyrimidyl, 2- oder 3-Pyrazinyl oder 2- oder 3-Furanyl.

Sofern R¹ H bedeutet, weist R² bevorzugt die Bedeutung Hal, CN oder Alkyl mit 1 bis 7 C-Atomen, insbesondere aber Methyl, Ethyl auf. Sofern R² H bedeutet, weist R¹ bevorzugt die Bedeutung (CH₂)ₙCO₂R⁵, (CH₂)ₙCO-Het, CHO, CH₂OR⁵, (CH₂)ₙ-Het. (CH₂)ₙN(R⁵)₂ oder CH=N-OA, insbesondere aber (CH₂)ₙCO₂R⁵, (CH₂)ₙCO-Het, CHO, CH=N-OA oder (CH₂)ₙ-Het auf. Besonders bevorzugt bedeutet R² H.

Weitere bevorzugte Bedeutungen von R¹ und R² ergeben sich aus den Beispielen.

R⁴ bedeutet bevorzugt H, Hal, CN, A oder NO₂, insbesondere H oder Hat.

R⁵ weist vorzugsweise die Bedeutung A auf.

R⁶ bedeutet bevorzugt (CH₂)ₙAr, insbesondere Ar.

A bedeutet bevorzugt Alkyl, ist vorzugsweise unverzweigt und hat 1, 2, 3, 4, 5, 6, 7, 8, 9 oder 10 C-Atome, vorzugsweise 1, 2, 3, 4, 5 oder 6 C-Atome und bedeutet vorzugsweise Methyl, Ethyl, n-oder Propyl, weiterhin bevorzugt Isopropyl, Butyl, Isobutyl, sek.-Butyl oder tert.-Butyl, aber auch n-Pentyl, neo-Pentyl, Isopentyl oder n-Hexyl. Besonders bevorzugt ist Methyl, Ethyl, n-Propyl, Isopropyl, n-Butyl, n-Pentyl, n-Hexyl oder n-Decyl -

Ferner weist A bevorzugt die Bedeutung der Gruppe (CH₂)ₘOCH₃ oder (CH₂)ₘC₂H₅ auf, worin m 2,3. 4, 5 oder 6, insbesondere aber 2 bedeutet.

Sofern A Alkenyl bedeutet, steht es vorzugsweise für Allyl, 2- oder 3-Butenyl, Isobutenyl, sek.-Butenyl, ferner bevorzugt ist 4-Pentenyl, iso-Pentenyl oder 5-Hexenyl.

Het ist vorzugsweise ein unsubstituierter oder durch A substituierter aromatischer und insbesondere gesättigter heterocyclischer Rest. Bevorzugt bedeutet Het 1-Piperidyl, 1-Piperazyl, 1-(4-Methyl)-piperazyl, 1-(4-Ethyl)-piperazinyl, 1-(4-Cyclopentyl)-piperazinyl, 4-Methylpiperazin-1-ylamin, 1-Pyrrolidinyl, 1-Pyrazolidinyl 1-(2-Methyl)-pyrazolidinyl, 1-Imidazolidinyl oder 1-(3-Methyl)-imidazolidinyl oder 4-Pyridyl, das unsubstituiert oder durch eine oder mehrere CN-Gruppe substituiert sein kann, 2- oder 4-Pyridazyl, 2-, 4- oder 5-Pyrimidyl, 2- oder 3-Pyrazinyl. Weiterhin bedeutet Het bevorzugt einen Rest aus der folgenden Tabelle:

| | |
|---|---|
| 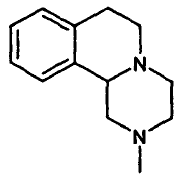 | 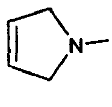 |
| 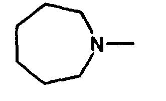 | 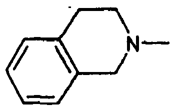 |
| 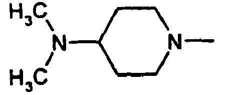 | 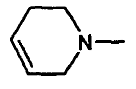 |
| 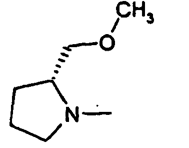 | 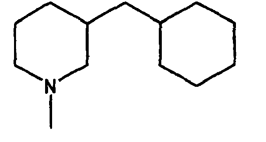 |
| 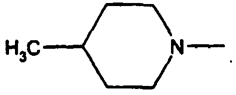 | 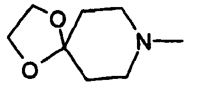 |
| 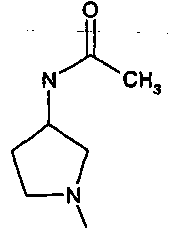 | 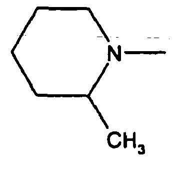 |
| 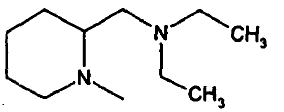 | 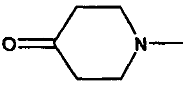 |
| 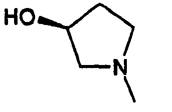 | 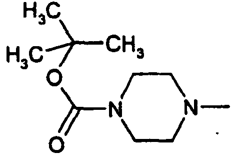 |
| 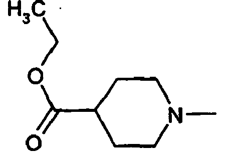 | 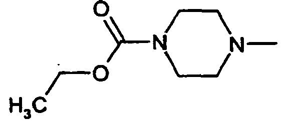 |
| 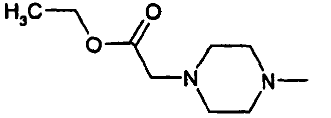 | 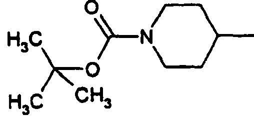 |
| 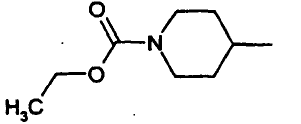 | 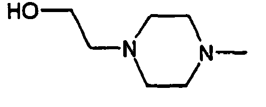 |
| 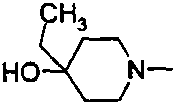 | 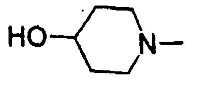 |
| 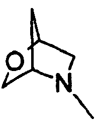 | 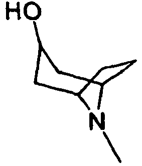 |
| 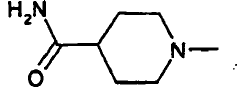 | 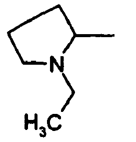 |
| 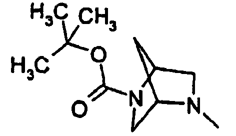 | 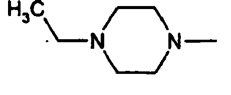 |
| 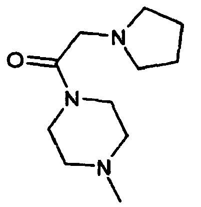 | 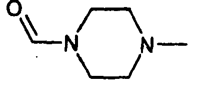 |
| 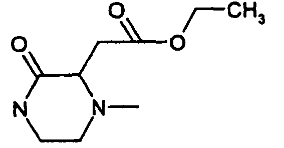 | 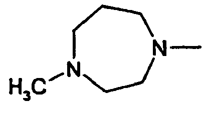 |
| 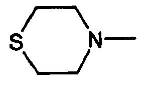 | 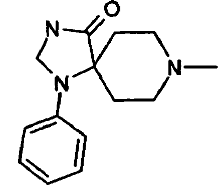 |
| 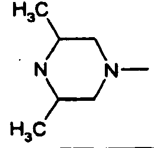 | 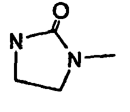 |
| 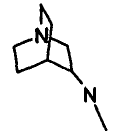 | 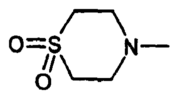 |
| 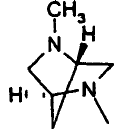 | 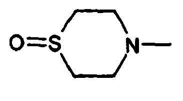 |
| 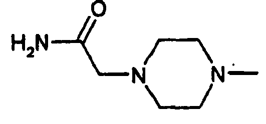 | 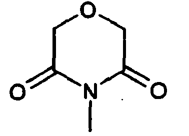 |
| 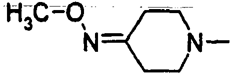 | 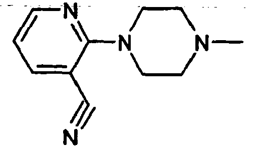 |
| 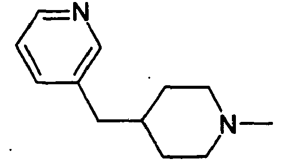 | 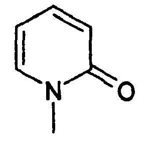 |
| 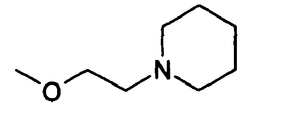 | 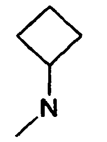 |
| 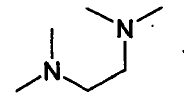 | 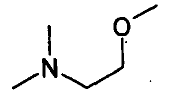 |
| 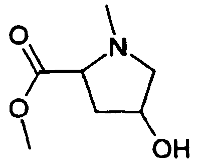 | 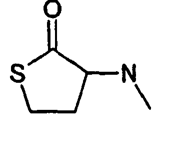 |
| 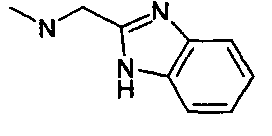 | 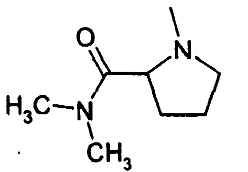 |
| 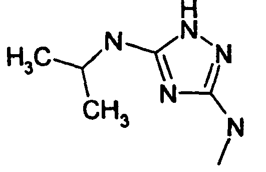 | 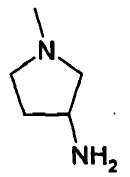 |
| 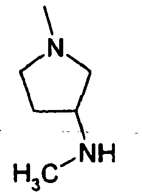 | 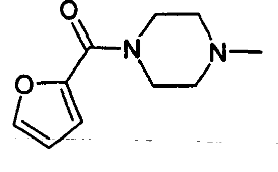 |
| 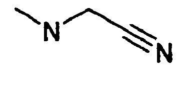 | 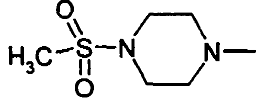 |
| 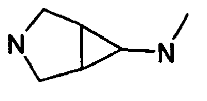 | 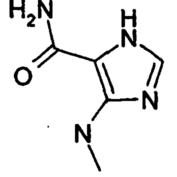 |
| 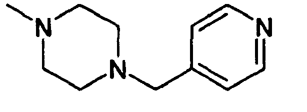 | 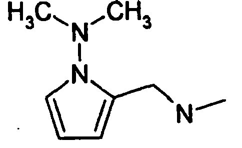 |
| 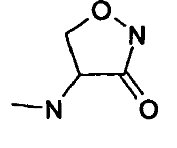 | 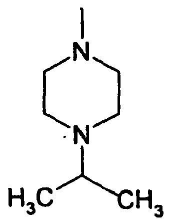 |
| 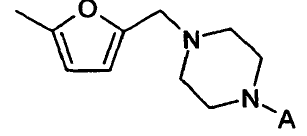 | 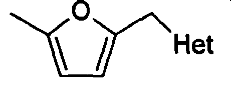 |

Besonders bevorzugt bedeutet Het einen der folgenden Reste:

| | | | | | |
|---|---|---|---|---|---|
| 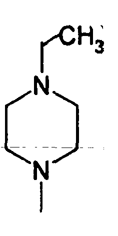 | 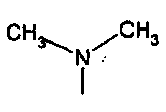 | 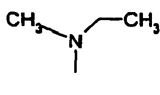 | 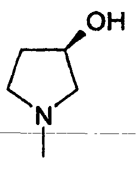 | 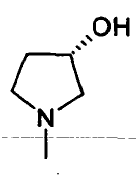 | 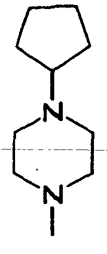 |
| 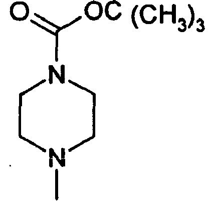 | | 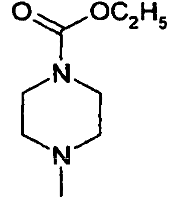 | | 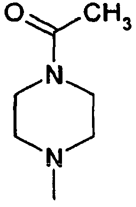 | |

Ar bedeutet vorzugsweise einen unsubstituierten oder durch Hal, OH, CN, NO₂, NH₂, NHCOCH₃, COOCH₃ CONH₂ oder CF₃ substituierten Phenylrest. Vorzugsweise ist Ar in 4- oder 3-Position substituiert.

n bedeutet vorzugsweise 0, 1 oder 2, insbesondere 0 oder 1.

Cycloalkyl hat vorzugsweise 3-7 C-Atome und steht bevorzugt für Cyclopropyl und Cyclobutyl, weiterhin bevorzugt für Cyclopentyl oder Cyclohexyl, ferner auch für Cycloheptyl, besonders bevorzugt ist Cyclopentyl.

Hal bedeutet vorzugsweise F, Cl oder Br, aber auch I.

Sofern die Verbindungen der Formel I ein oder mehrere chirale C-Atome aufweist, sind die Enantiomeren, Diastereomere und deren Mischungen Gegenstand der vorliegenden Erfindung.

Für die gesamte Erfindung gilt, daß sämtliche Reste, die mehrfach auftreten, gleich oder verschieden sein können, d.h. unabhängig voneinander sind.

Dementsprechend sind Gegenstand der Erfindung insbesondere diejenigen Verbindungen der Formel I, in denen mindestens einer der genannten Reste eine der vorstehend angegebenen bevorzugten Bedeutungen hat.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart), beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.
Die Verbindung der Formel 111 wird vorzugsweise durch Umsetzung von Verbindungen der Formel V 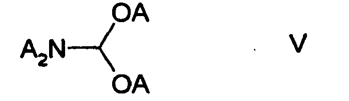 worin A die oben angegebene Bedeutung aufweist, mit Verbindungen der Formel VI 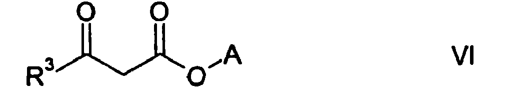 worin R³ und A die oben angegebene Bedeutung aufweisen,
unter für derartige Reaktionen bekannten Bedingungen erhalten.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.
Andererseits ist es möglich, die Reaktion stufenweise durchzuführen.

Die Ausgangsstoffe der Formeln II, III und IV sind in der Regel bekannt. Sofern sie nicht bekannt sind, können sie nach an sich bekannten Methoden hergestellt werden.

Im einzelnen erfolgen die Umsetzungen der Verbindungen der Formel II mit den Verbindungen der Formel III und den Verbindungen der Formel IV in Gegenwart oder Abwesenheit eines vorzugsweise inerten Lösungsmittels bei Temperaturen zwischen etwa -20 und etwa 150°, vorzugsweise zwischen 20 und 100°.

Als inerte Lösungsmittel eignen sich z.B. Kohlenwasserstoffe wie Hexan, Petrolether, Benzol, Toluol oder Xylol; chlorierte Kohlenwassertoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Nitroverbindungen wie Nitromethan oder Nitrobenzol; Ester wie Ethylacetat oder Gemische der genannten Lösungsmittel.

Der für die Umsetzung erforderliche pH-Wert kann in Anlehnung an für ähnliche Umsetzungen von Carbonyl- mit Aminoverbindungen gewählte pH-Werte eingestellt werden. Vorzugsweise wird der pH-Wert durch die Verwendung des jeweiligen Säureadditionssalzes vorzugsweise eines Halogenwasserstoff-Additionssalzes der Verbindung der Formel II vorgegeben, d.h. es erfolgt keine zusätzliche Basen- oder Säurezugabe zur Reaktionsmichung. Bevorzugte Säureadditionssalze sind Hydrochloride oder -bromide

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B.

Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure; 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und /oder Aufreinigung der Verbindungen der Formel 1 verwendet werden.

Andererseits können, falls gewünscht, die freien Basen der Formel I aus ihren Salzen mit Basen (z.B. Natrium- oder Kaliumhydroxid oder -carbonat) in Freiheit gesetzt werden.

Gegenstand der Erfindung sind insbesondere Verbindungen der Formel 1 und ihre physiologisch unbedenklichen Salze und Solvate als Arzneimittel .

Gegenstand der Erfindung sind auch die Verbindungen der Formel 1 und ihre physiologisch unbedenklichen Salze und Solvate als Glycin-Transporter-Inhibitoren.

Gegenstand der Erfindung ist ferner die Verwendung der Verbindungen der Formel I und/oder ihrer physiologisch unbedenklichen Salze und/oder Solvate zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner pharmazeutische Zubereitungen, enthaltend mindestens eine Verbindung der Formel I und/oder eines ihrer physiologisch unbedenklichen Salze und/oder Solvate.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen' Verbindungen nicht reagieren, beispielsweise Wasser, pflanzliche Öle, Benzylalkohole, Alkylenglykole, Polyethylenglykole, Glycerintriacetat, Gelatine, Kohlenhydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder ein oder mehrere weitere Wirkstoffe enthalten, z.B. ein oder mehrere Vitamine.

Dabei werden die erfindungsgemäßen Substanzen in der Regel vorzugsweise in Dosierungen zwischen 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit. Arzneistoffkombination und Schwere der jeweiligen Erkrankung, welcher die Therapie gilt. Die orale Applikation ist bevorzugt.

Bevorzugte Verbindungen der Formel 1 weisen nanomolare Affinität zu den 5 HT2A-Rezeptoren auf. Besonders bevorzugte Verbindungen der Formel I weisen eine geringe Affinität zum 5 HT2C-Rezeptor auf. Ganz besonders bevorzugte Verbindungen der Formel I zeigen keine signifikante Glycin-Transporter-Aktivität.

Vor- und nachstehend sind alle Temperaturen in °C angegeben. In den nachfolgenden Beispielen,bedeutet "übliche Aufarbeitung": Man gibt, falls erforderlich, Wasser hinzu, extrahiert mit Ethylacetat oder Dichlormethan, trennt ab, trocknet die organische Phase über Natriumsulfat, dampft ein und reinigt durch Chromatographie an Kieselgel und /oder durch Kristallisation.

### Beispiel 1

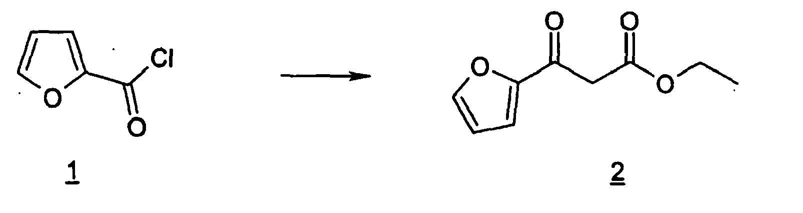

In einem 6 I Dreihalskolben, versehen mit Rührer, Kühler, Thermometer, Tropftrichter und Trockenröhrchen, werden 130 g Monoethylmalonat Kaliumsalz in 21 Ethylacetat suspendiert, unter Kühlen und Rühren bei 0°C mit 127 ml Triethylamin und 82,4 g Magnesiumchlorid (wasserfrei) versetzt und langsam auf 35-40°C erwärmt. Bei dieser Temperatur wird 6 h nachgerührt, wieder auf 0°C abgekühlt und unter Kühlen und Rühren bei 0°C eine Lösung von 50 ml Furan-2-carbonsäurechlorid in 1 I Ethylacetat innerhalb von 15 Minuten zugetropft. Es wird über Nacht bei RT nachgerührt, dann unter Kühlen und Rühren 1,21 13%ige Salzsäure zugetropft und die Ethylacetatphase abgetrennt. Nach üblicher Aufarbeitung erhält man das Produkt 2 als leicht gelbliche Flüssigkeit. (Sp. 85°C/0,6-0,5 mbar).

### Beispiel 2

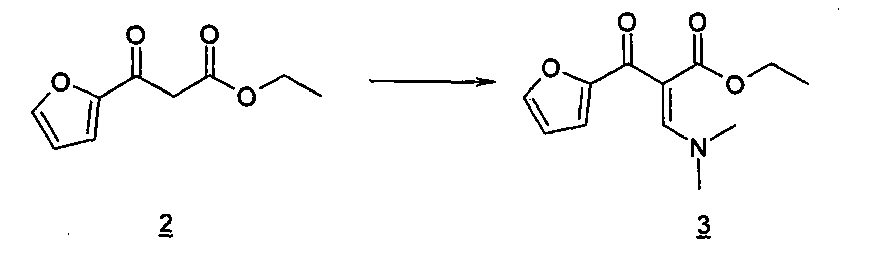

In einem 250 ml Kolben, versehen mit Magnetrührer, Kühler und Trockenröhrchen, werden 5 g Ethyl-2-furoylacetat in 100 ml THF, abs. gelöst, mit 7,4 ml N,N-Dimethylformamiddimethylacetal versetzt und 6 h am Rückfluß gerührt. Dann wurde die Reaktionslösung zum Rückstand 6,38 g (100%) abgezogen wodurch 3 erhalten wird.

### Beispiel 3

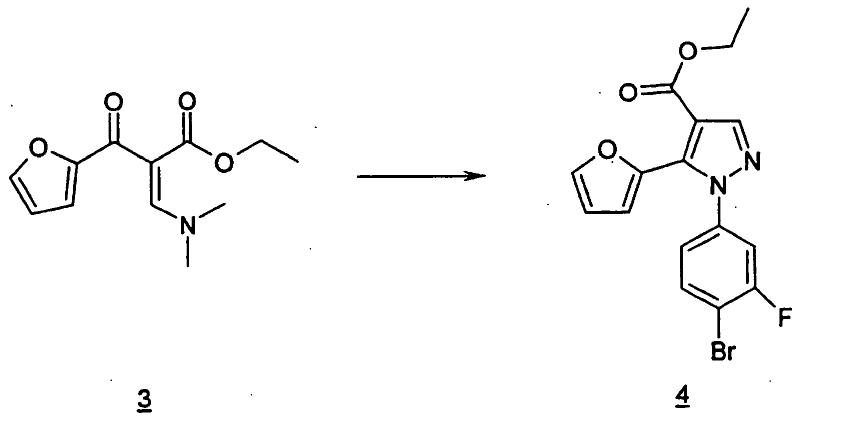

In einem 250 ml Einhalskolben, versehen mit Magnetrührer, Kühler und Trockenröhrchen, werden 4,49 g des beta-Ketoesters 3 in 90 ml abs. Ethanol gelöst, mit 4,2 g 4-Bromphenylhydraziniumchlorid versetzt und über Nacht am Rückfluß gerührt. Durch übliche Aufarbeitung wird 4 erhalten.

### Beispiel 4

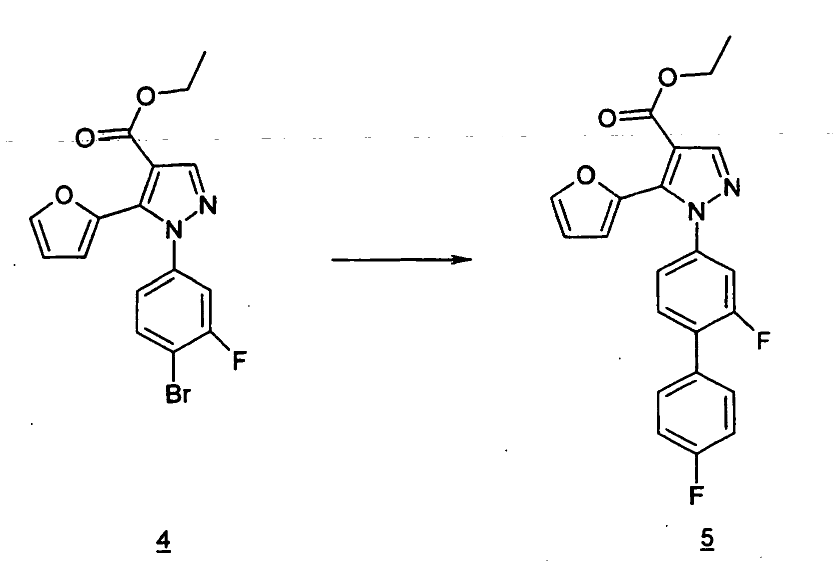

2,00 g des Arylbromids 4 und 0,203 g [1,1'-Bis(diphenylphosphino)ferocene)dichlor-palladium II werden nacheinander in 80 ml Dimethoxyethan gelöst, 1,40 g 4-Fluorphenylboronsäure zugegeben und anschließend eine Lösung von Na₂CO₃ in Wasser (5,87 g in 25 ml) zugegeben. Die Reaktionslösung rührt über Nacht bei RT. Zur Aufarbeitung wirde der Reaktionsansatz zwischen Diethylether und Wasser verteilt. Nach üblicher Aufarbeitung wird 5 erhalten.

### Beispiel 5

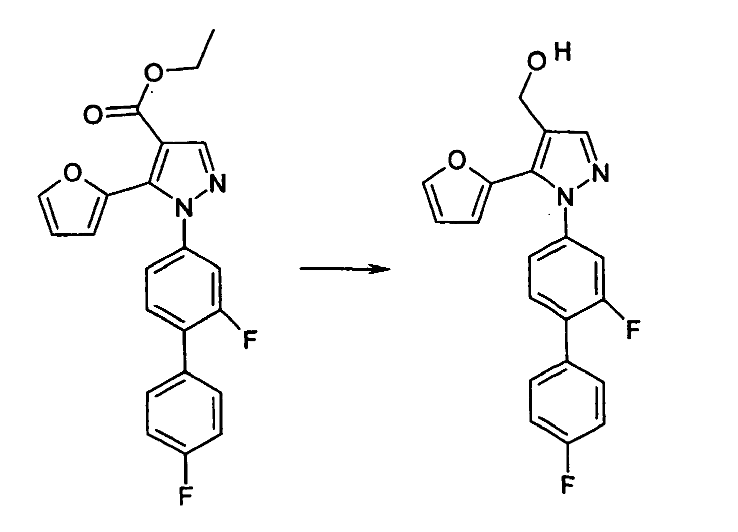

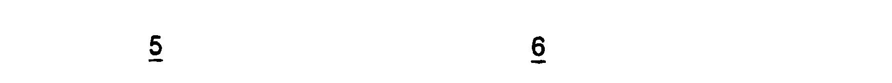

1,60 g des Esters 5 werden in THF vorgelegt auf ca. 5 bis 0°C gekühlt und anschließend 4,3 ml einer 1 M Lösung von LiAlH₄ in THF langsam zugetropft. Nach vollständiger Zugabe rührt man über Nacht bei Raumtemeratur weiter. Durch übliche Aufarbeitung wird 6 kristallner Feststoff erhalten.

### Beispiel 6

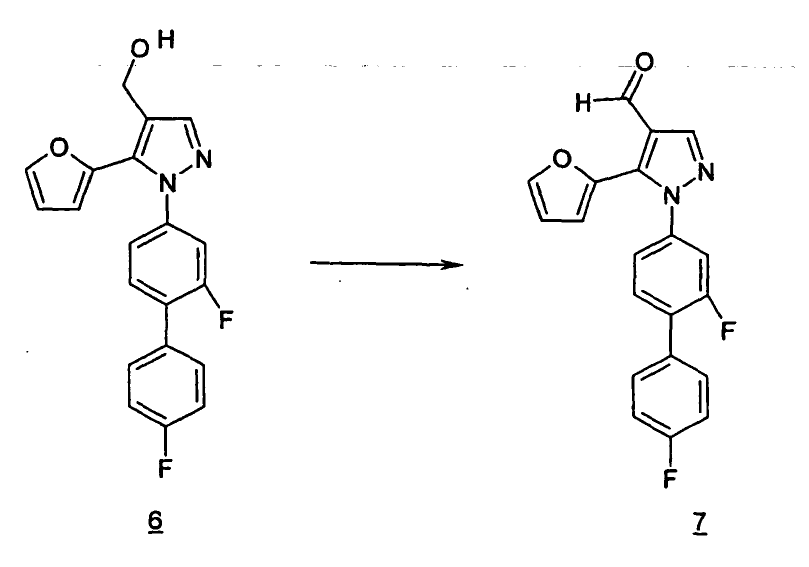

1,4 g des Alkohols 6 wird in einer Mischung aus 10 ml THF und 40 ml Dichlormethan gelöst. Anschließend werden 2,62 g Mangandioxid zugegeben und der Reaktionsansatz über Nacht bei RT gerührt. Durch übliche Aufarbeitung wird das Produkt 7 als kristallner Feststoff erhalten.

### Beispiel 7

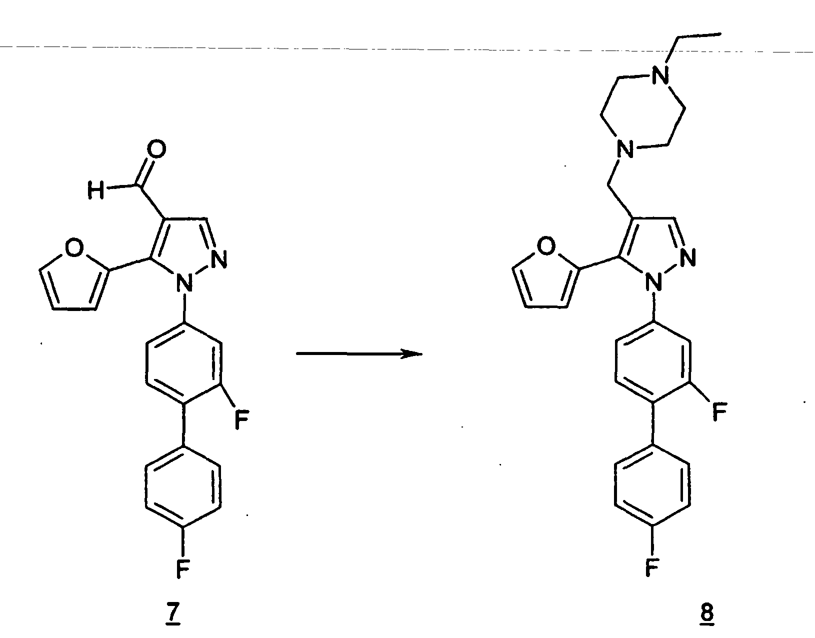

Zu einer Mischung von 200 mg des Aldehyds 7, 103 mg Ethylpiperazin, 3,6 ml 1,2-Dichlorethan und 1,8 ml THF werden, 36 µl CH₃COOH gegeben.
Die Mischung wird für 3 h bei Raumtemperatur gerührt. Anschließend werden 0,23 g NaB(OAc)₃H zugegeben und 16 h weiter gerührt.

Durch übliche Aufarbeitung wird 1-Ethyl-4-[1-(4'-fluoro-biphenyl-4-yl)-5-furan-2-yl-1H-pyrazol-4-yl-methyl]-Piperazinedihydrochlorid 8 als farbloser Feststoff erhalten.

Unter Verwendung der entsprechenden Vorstufen werden in analoger Weise die folgenden Verbindungen der Formel I erhalten:

### Beispiele 8 - 51:

| | |
|---|---|
| (8) | 1-[1-(3,4'-Difluoro-biphenyl-4-yl)-5-furan-2-yl-1 H-pyrazol-4-ylmethyl]-4-methyl-piperazine |
| (9) | 1-[1-(3,4'-Difluoro-biphenyl-4-yl)-5-furan-2-yl-1H-pyrazo-4-ylmethyl]-4-ethyl-piperazine |
| (10) | 1-[1-(3,4'-Ditluoro-biphenyl-4-yl)-5-furan-2-yl-1 H-pyrazol-4-ylmethyl]-pyrrolidin-3-ol |
| (11) | 1-[1-(3,4'-Difluoro-biphenyl-4-yl)-5-furan-2-yl-1 H-pyrazol-4-ylmethyl]-piperazine |
| (12) | (1 -(3,4'-Difluoro-biphenyl-4-yl)-5-furan-2-yl-1 H-pyrazol-4-ylmethyl)-dimethyl-amine |
| (13) | [1-(3,4'-Difluoro-biphenyl-4-yl)-5-furan-2-yl-1H-pyrazol-4-ylmethyl]-ethyl-methyl-amine |
| (14) | [1-(3,4'-Difluoro-biphenyl-4-yl)-5-furan-2-yl-1 H-pyrazol-4-ylmethyl]-methyl-(1-methyl-pyrrolidin-3-yl)-amine |
| (15) | 1-[1-(2,4'-Difluorbiphenyl-4-yI)-5-furan-2-yl-1 H-pyrazol-4-ylmethyl]-4-methylpiperazin |
| (16) | [1-(2,4'-Difluorbiphenyl-4-yl)-5-furan-2-yl-1 H-pyrazol-4-ylmethyl]-ethylmethylamin |
| (17) | 1-[1-(4'-Fluoro-biphenyl-4-yl)-3-methyl-5-phenyl-1H-pyrazol-4-ylmethyl]-4-methyl-piperazine |
| (18) | Ethyl-[1-(4'-fluoro-biphenyl-4-yl)-3-methyl-5-phenyl-1 H-pyrazol-4-ylmethyl]-methyl-amine |
| (19) | (1-Biphenyl-4-yl-3-methyl-5-phenyl-1H-pyrazol-4-yl)-acetic acid ethyl ester |
| (20) | 2-(1-Biphenyl-4-yl-3-methyl-5-phenyl-1H-pyrazol-4-yl)-ethanol |
| (21) | 1-{5-[2-(4'-Fluoro-biphenyl-4-yl)-5-methyl-2H-pyrazol-3-yl]-furan-2-ylmethyl}-4-methyl-piperazine |
| (22) | 1-Ethyl-4-{5-[2-(4'-fluoro-biphenyl-4-yl)-5-methyl-2H-pyrazol-3-yl]- , furan-2-ylmethyl}-piperazine |
| (23) | Ethyl-{5-[2-(4'-fluoro-biphenyl-4-yl)-5-methyl-2H-pyrazol-3-yl]-furan-2-ylmethyl}-methyl-amine |
| (24) | 1-{5-[2-(4'-Fluoro-biphenyl-4-yl)-5-methyl-2H-pyrazol-3-yl]-furan-2-ylmethyl}-pyrrolidin-3-ol |
| (25) | 1-[1-(4'-Fluorbiphenyl-4-yl)-3,5-dimethyl-1H-pyrazol-4-ylmethyl]-4-methylpiperazin , |
| (26) | 1-Ethyl-4-[1-(4'-fluorbiphenyl-4-yl)-3,5-dimethyl-1 H-pyrazol-4-ylmethyl]-piperazin |
| (27) | Ethyl-[1-(4fluorbiphenyl-4-yl)-3,5-dimethyl-1H-pyrazol-4-ylmethyl]-methylamin |
| (28) | 1-[1-(4'-Fluorbipheny)-4-yl)-5-methyl-1H-pyrazom-4-ylmethyl]-4-methylpiperazin |
| (29) | 1-Ethyl-4-[1-(4'-fluorbiphenyl-4-yl)-5-methyl-1H-pyrazol-4-ylmethyl]-piperazin |
| (30) | 1-[1-(4'-Fluorbiphenyl-4-yl)-5-methyl-1H-pyrazol-4-ylmethyl]-pyrrolidin-3-ol |
| (31) | Ethyl-[1-(4'-fluorbiphenyl-4-yl)-5-methyl-1H-pyrazol-4-ylmethyl]-methylamin |
| (32) | 1-[1-(4'-Fluorbiphenyl-4-yl)-3,5-dimethyl-1H-pyrazol-4-ylmethyl]-pyrrolidin-3-ol |
| (33) | 1-[1-(4'-Fluoro-biphenyl-4-yl)-1H-pyrazol-4-ylmethyll-pyrrolidin-3-ol |
| (34) | 1-[1-(4'-Fluoro-biphenyl-4-yl)-1H-pyrazol-4-ylmethyl]-4-methyl-piperazine |
| (35) | 1-Ethyl-4-[1-(4'-fluoro-biphenyl-4-yl)-1H-pyrazol-4-ylmethyl]-piperazine |
| (36) | 1-[1-(4'-Fluor-2-methylbiphenyl-4-yl)-5-furan-2-yl-1H-pyrazol-4-ylmethyl]-4-methylpiperazin |
| (37) | 1-Ethyl-4-[1-(4'-fluor-2-methylbiphenyl-4-yl)-5-furan-2-yl-1H-pyrazol-4-ylmethyl]-piperazin |
| (38) | Ethyl-[1-(4'-fluor-2-methylbiphenyl-4-yl)-5-furan-2-yl-1H-pyrazol-4-ylmethyl]-methylamin |
| (39) | 1-[1-(4'-Fluor-2-methylbiphenyl-4-yl)-5-furan-2-yl-1 H-pyrazol-4-ylmethyl]-pyrrolidin-3-ol |
| (40) | 1-[1-(2-Chlor-4'-fluorbiphenyl-4-yl)-5-furan-2-yl-1H-pyrazol-4-ylmethyl]-4-methylpiperazin |
| (41) | 1-[1-(2-Chlor-4'-fluorbiphenyl-4-yl)-5-furan-2-yl-1 H-pyrazol-4-ylmethyl]-4-ethylpiperazin |
| (42) | 1-[1-(2-Chlor-.4'-tluorbiphenyl-4-yl)-5-furan-2-yl-1 H-pyrazol-4-ylmethyl]-pyrrolidin-3-ol |
| (43) | [1-(2-Chloo-4'-fluorbiphenyl-4-yl)-5-furan-2-yl-1H-pyrazol-4- |
| | ylmethyl]-ethylmethylamin |
| (44) | 1-[1-(4'-Fluor-3-methylbiphenyl-4-yl)-5-furan-2-yl-1 H-pyrazol-4-ylmethyl]-4-methylpiperazin |
| (45) | 1-Ethyl-4-[1-(4'-fluor-3-methylbiphenyl-4-yl)-5-furan-2-yl-1H-pyrazol-4-ylmethyl]-piperazin |
| (46) | 1-[1-(4'-Fluor-3-methylbiphenyl-4-yl)-5-furan-2-yl-1H-pyrazol-4-ylmethyl]-pyrrolidin-3-ol |
| (47) | Ethyl-[1-(4'-fluor-3-methylbiphenyl-4-yl)-5-furan-2-yl-1 H-pyrazol-4-ylmethyl]-methylamin |
| (48) | 1-[5-Furan-2-yl-1-(2,6,4'-trifluorbiphenyl-4-yl)-1 H-pyrazol-4-ylmethyl]-4-methylpiperazin |
| (49) | 1-Ethyl-4-[5-furan-2-yl-1-(2,6,4'-trifluorbiphenyl-4-yl)-1 H-pyrazol-4-ylmethyl]-piperazin |
| (50) | 1-[5-Furan-2-yl-1-(2,6,4'-trifluorbiphenyl-4-yl)-1 H-pyrazol-4-ylmethyl]-pyrrolidin-3-ol |
| (51) | Ethyl-[5-furan-2-yl-1-(2,6,4'-trifluorbiphenyl-4-yl)-1H-pyrazol-4-ylmethyl]-methylamin |

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel 1 mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄·2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel 1, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g Wirkstoff der Formel I in 10 isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Verbindungen der Formel I 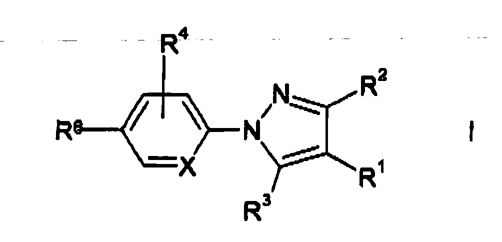 worin
R², R⁴ H, A, Hal, Cycloalkyl mit 3 bis 7 C-Atomen, CF₃, NO₂, CN, OCF₃, OA, NHA, NA₂, NH₂,
R³, R⁶ (CH₂)ₙHet, (CH₂)ₙAr,
R¹ H oder ein organischer Rest, insbesondere (CH₂)ₙCO₂R⁵, (CH₂)ₙCOHet, CHO, (CH₂)ₙOR⁵, (CH₂)ₙHet, (CH₂)ₙN(R⁵)₂, CH=N-OA, CH₂CH=N-OA, (CH₂)ₙNHOA, (CH₂)ₙ(R⁵)Het, (CH₂)ₙCH=N-Het, (CH₂)ₙOCOR⁵, (CH₂)ₙN(R⁵)CH₂CH₂OR⁵, (CH₂)ₙN(R⁵)CH₂CH₂OCF₃, (CH₂)ₙN(R⁵)C(R⁵)COOR⁵, (CH₂)ₙN(R⁵)CH₂COHet, (CH₂)ₙN(R⁵)CH₂Het, (CH₂)ₙN(R⁵)CH₂CH₂Het, (CH₂)ₙN(R⁶)CH₂CH₂N(R⁵)CH₂COOR⁵, (CH₂)ₙN(R⁵)CH₂CH₂N(R⁵)₂, CH=CHCOOR⁵, CH=CHCH₂NR⁵Het, CH=CHCH₂N(R⁵)₂, CH=CHCH₂OR⁵ oder (CH₂)ₙN(R⁵)Ar,
R⁵ H oder A
A geradkettiges oder verzweigtes Alkyl oder Alkoxy mit 1 bis 10 C-Atomen, Alkenyl oder Alkenyloxyalkyl mit 2 bis 10 C-Atomen,
Het einen unsubstituierten oder einfach oder mehrfach durch A und/oder Hal substituierten, gesättigten, ungesättigten oder aromatischen mono- oder bicyclischen heterocyclischen ein oder mehrere Heteroatome enthaltenden organischen Rest,
Ar einen unsubstituierten oder einfach oder mehrfach durch A und/oder Hal, OR⁵, OOCR⁵, COOR⁵, CON(R⁵)₂, CN, NO₂, NH₂, NHCOR⁵, CF₃ oder SO₂CH₃ substituierten Phenylrest,
n 0, 1, 2, 3, 4 oder 5
und
Hal F, Cl, Br oder I
bedeuten
sowie
X CH oder N, wobei im Fall dass X die Bedeutung CH aufweist, R² und R⁴ nicht gleichzeitig H bedeuten,
sowie deren Salze und Solvate, Enantiomere, Racemate und andere Mischungen der Enantiomere, insbesondere deren physiologisch verträglichen Salze und Solvate.

2. Verbindungen der Formel I nach Anspruch 1, worin R⁶ Phenyl, 2-, 3-oder 4-Cyanophenyl, 2-, 3- oder 4-Fluorphenyl, 2-, 3- oder 4-Methyl-,

3. Verbindungen der Formel I nach einem oder mehreren der vorhergehenden Ansprüche, worin R⁴ H, Hal, CN, A oder N0₂ bedeutet.

4. Verbindungen der Formel I nach einem oder mehreren der vorhergehenden Ansprüche, worin R² H oder Alkyl bedeutet.

5. Verbindungen der Formel I nach einem oder mehreren der vorhergehenden Ansprüche, worin R³ Phenyl, 2-, 3- oder 4-Cyanophenyl, 2-, 3- oder 4-Fluorphenyl, 2-, 3- oder 4-Methyl-, Ethyl-, n-Propyl- oder n-Butylphenyl, 2,3-, 2,4-, 2,5-, 2,6-Difluor- oder Dicyanophenyl, Thiophen-2-yl oder Thiophen-3-yl, 2-, 3- oder 4-Pyridyl, 2-, 4- oder 5-Oxazolyl, 2-, 4- oder 5-Thiazolyl, Chinolinyl, Isochinolinyl, 2- oder 4-Pyridazyl, 2-, 4- oder 5-Pyrimidyl, 2- oder 3-Pyrazinyl oder 2- oder 3-Furanyl bedeutet.

6. Verbindungen der Formel I nach einem oder mehreren der vorhergehenden Ansprüche, worin X die Bedeutung N aufweist.

7. Verbindungen der Formeln IA, IB, IC, ID, IE und IF: 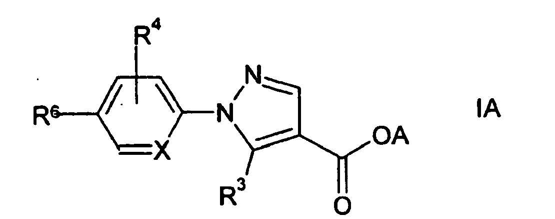 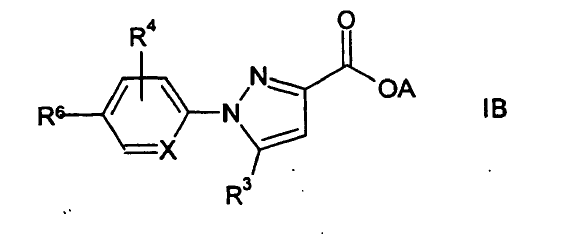 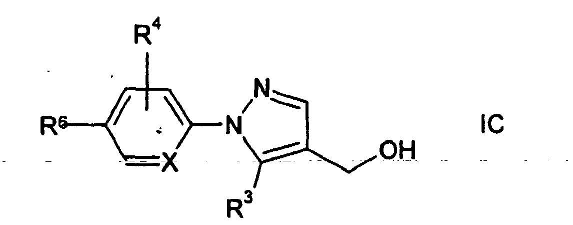 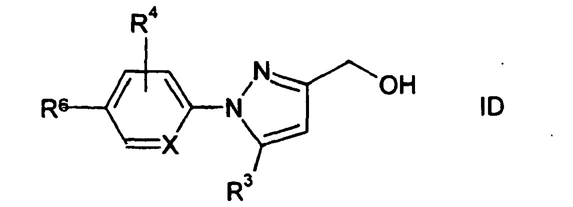 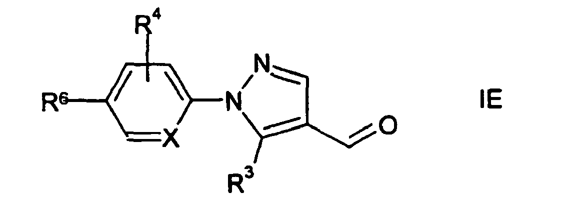 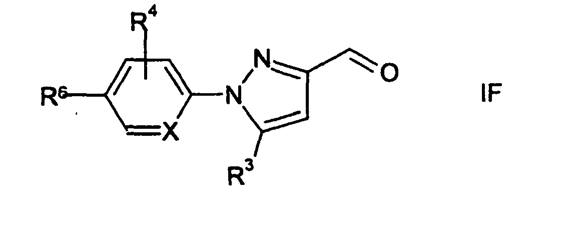 worin
R³, R⁴, R⁶ und X die in Anspruch 1 angegebenen Bedeutungen aufweisen.

8. Verfahren zur Herstellung von Verbindungen der Formel IA 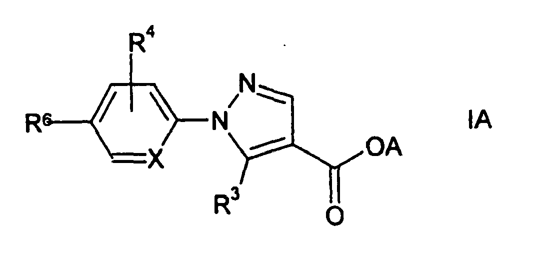 worin R³, R⁴, R⁶, X und A die in Anspruch 1 angegebene Bedeutung aufweisen
sowie deren Salze und Solvate, das **dadurch gekennzeichnet ist, daß** man eine Verbindung der Formel II 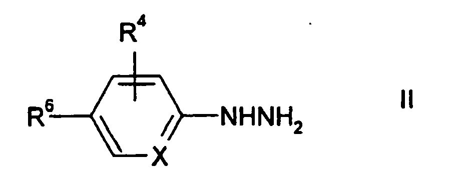 oder deren Säureadditionssalze
worin
R⁴, R⁶ und X die in Anspruch 1 angegebenen Bedeutungen haben, mit einer Verbindung der Formel III 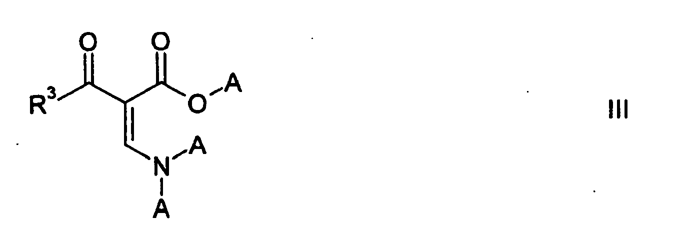 worin
A und R³ die in Anspruch 1 angegebenen Bedeutungen haben, umsetzt
und/oder daß man eine basische Verbindung der Formel IA durch Behandeln mit einer Säure in eines ihrer Salze überführt.

9. Verfahren zur Herstellung von Verbindungen der Formel IB 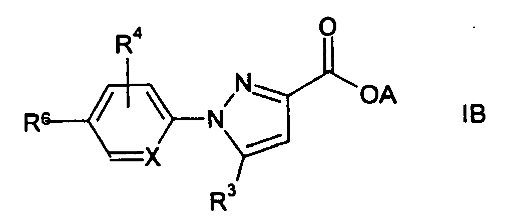 worin R³, R⁴, R⁶, X und A die in Anspruch 1 angegebene Bedeutung aufweisen
sowie deren Salze und Solvate, das **dadurch gekennzeichnet ist, daß** man eine Verbindung der Formel II 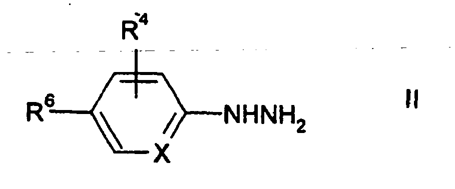 oder deren Säureadditionssalze
worin
R⁴, R⁶ und X die in Anspruch 1 angegebenen Bedeutungen haben, mit einer Verbindung der Formel IV 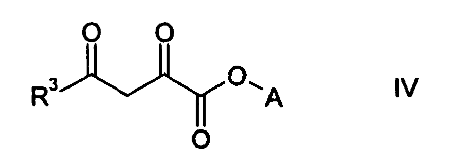 worin
A und R³ die in Anspruch 1 angegebenen Bedeutungen haben, umsetzt
und/oder daß man eine basische Verbindung der Formel IB durch Behandeln mit einer Säure in eines ihrer Salze überführt.

10. Verbindungen der Formel I nach einem oder mehreren der Ansprüche 1 bis 6 und ihre physiologisch unbedenklichen Salze und Solvate als Arzneimittel.

11. Verwendung der Verbindungen der Formel I nach einem oder mehreren der Ansprüche 1 bis 6 , sowie deren Salze und Solvate, zur Herstellung eines Arzneimittels zur Behandlung und Prophylaxe von Krankheiten, die durch die Bindung der Verbindungen der Formel I an 5 HT-Rezeptoren beeinflusst werden können.

12. Verwendung von Verbindungen der Formel I nach einem oder mehreren der Ansprüche 1 bis 6 und/oder deren physiologisch unbedenkliche Salze und Solvate zur Herstellung eines Arzneimittels mit 5-HT-Rezeptor-antagonistischer Wirkung.

13. Verwendung von Verbindungen der Formel 1 nach einem oder mehreren der Ansprüche 1 bis 6 und/oder deren physiologisch unbedenkliche Salze und Solvate zur Herstellung eines Arzneimittels mit 5-HT2A-Rezeptor-antagonistischer Wirkung.

14. Pharmazeutische Zubereitung, **gekennzeichnet durch** einen Gehalt an mindestens einer Verbindung der Formel I nach einem oder mehreren der Ansprüche 1 bis 6 und/oder einem ihrer physiologisch unbedenklichen Salze und/oder eines ihrer Solvate.

15. Verfahren zur Herstellung pharmazeutischer Zubereitungen, **dadurch gekennzeichnet, daß** man eine Verbindung der Formel I nach Anspruch 1 und/oder eines ihrer physiologischen unbedenklichen Salze und/oder eines ihrer Solvate zusammen mit mindestens einem festen, flüssigen oder halbflüssigen Träger- oder Hilfsstoff in eine geeignete Dosierungsform bringt.

16. Verwendung von Verbindungen der Formel nach einem oder mehreren der Ansprüche 1 bis 6 und/oder ihre physiologisch unbedenklichen Salze oder Solvate zur Herstellung eines Arzneimittels zur Prophylaxe und/oder Behandlung von Psychosen, neurologischen Störungen, amyotropher Lateralsklerose, Essstörungen wie Bulimie, nervöser Anorexie, des prämenstrualen Syndroms und/oder zur positiven Beeinflussung von Zwangsverhalten (obsessive-compulsive disorder, OCD).

17. Verbindungen der Formel I, worin R¹, R², R³, R⁴, R⁶ und X die in Anspruch 1 genannte Bedeutung aufweisen und Het in den Resten R¹, R³, und R⁶ einen der folgenden Reste bedeutet:
| | | | | | |
|---|---|---|---|---|---|
| 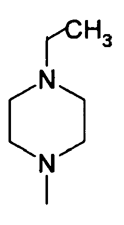 | 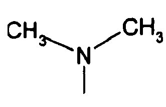 | 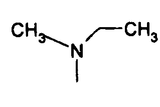 | 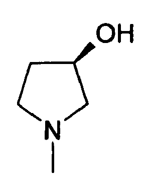 | 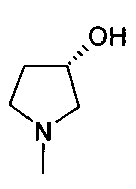 | 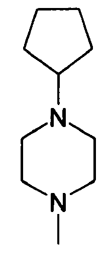 |
| 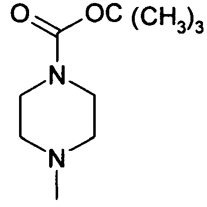 | | 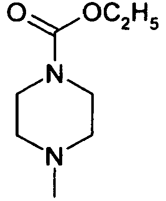 | | 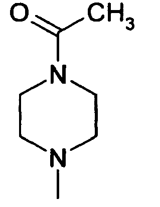 | |

## Claims

1. Compounds of the formula I 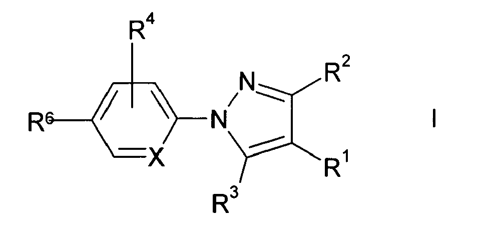 in which
R², R⁴ denote H, A, Hal, cycloalkyl having 3 to 7 C atoms, CF₃, NO₂, CN, OCF₃, OA, NHA, NA₂, NH₂,
R³, R⁶ denote (CH₂)ₙHet, (CH₂)ₙAr,
R¹ denotes H or an organic radical, in particular (CH₂)ₙCO₂R⁵, (CH₂)ₙCOHet, CHO, (CH₂)ₙOR⁵, (CH₂)ₙHet, (CH₂)ₙN(R⁵)₂, CH=N-OA, CH₂CH=N-OA, (CH₂)ₙNHOA, (CH₂)ₙ(R⁵)Het, (CH₂)ₙCH=N-Het, (CH₂)ₙOCOR⁵, (CH₂)ₙN(R⁵)CH₂CH₂OR⁵, (CH₂)ₙN(R⁵)CH₂CH₂OCF₃, (CH₂)ₙN(R⁵)C(R⁵)COOR⁵, (CH₂)ₙN(R⁵)CH₂COH et, (CH₂)ₙN(R⁵)CH₂Het, (CH₂)ₙN(R⁵)CH₂CH₂Het, (CH₂)ₙN(R⁵)CH₂CH₂N(R⁵)CH₂COOR⁵, (CH₂)ₙN(R⁵)CH₂CH₂N(R⁵)₂, CH=CHCOOR⁵, CH=CHCH₂NR⁵Het, CH=CHCH₂N(R⁵)₂, CH=CHCH₂OR⁵ or (CH₂)ₙN(R⁵)Ar,
R⁵ denotes H or A
A denotes straight-chain or branched alkyl or alkoxy having 1 to 10 C atoms, alkenyl or alkenyloxyalkyl having 2 to 10 C atoms,
Het denotes a saturated, unsaturated or aromatic mono- or bi-cyclic heterocyclic organic radical containing one or more heteroatoms which is unsubstituted or mono- or polysubstituted by A and/or Hal,
Ar denotes a phenyl radical which is unsubstituted or mono- or polysubstituted by A and/or Hal, OR⁵, OOCR⁵, COOR⁵, CON(R⁵)₂, CN, NO₂, NH₂, NHCOR⁵, CF₃ or SO₂CH₃,
n denotes 0, 1, 2, 3, 4 or 5
and
Hal denotes F, CI, Br or I
and
X denotes CH or N, where R² and R⁴ do not simultaneously denote H in the case where X has the meaning CH,
and their salts and solvates, enantiomers, racemates and other mixtures of the enantiomers, in particular their physiologically tolerated salts and solvates.

2. Compounds of the formula I according to Claim 1, in which R⁶ denotes phenyl, 2-, 3- or 4-cyanophenyl, 2-, 3- or 4-fluorophenyl, 2-, 3- or 4-methyl-, -ethyl-,-n-propyl- or -n-butylphenyl, 2,3-, 2,4-, 2,5-, 2,6-, 3,4-, 3,5- or 3,6-difluoro-, -dichloro- or -dicyanophenyl, 3,4,5-trifluorophenyl, 3,4,5-trimethoxy- or -triethoxyphenyl, thiophen-2-yl or thiophen-3-yl.

3. Compounds of the formula I according to one or more of the preceding claims, in which R⁴ denotes H, Hal, CN, A or NO₂.

4. Compounds of the formula I according to one or more of the preceding claims, in which R² denotes H or alkyl.

5. Compounds of the formula I according to one or more of the preceding claims, in which R³ denotes phenyl, 2-, 3- or 4-cyanophenyl, 2-, 3- or 4-fluorophenyl, 2-, 3- or 4-methyl-, -ethyl-, -n-propyl- or -n-butylphenyl, 2,3-, 2,4-, 2,5-, 2,6-difluoro- or -dicyanophenyl, thiophen-2-yl or thiophen-3-yl, 2-, 3- or 4-pyridyl, 2-, 4- or 5-oxazolyl, 2-, 4- or 5-thiazolyl, quinolinyl, isoquinolinyl, 2- or 4-pyridazyl, 2-, 4- or 5-pyrimidyl, 2- or 3-pyrazinyl or 2- or 3-furanyl.

6. Compounds of the formula I according to one or more of the preceding claims, in which X has the meaning N.

7. Compounds of the formulae IA, IB, IC, ID, IE and IF: 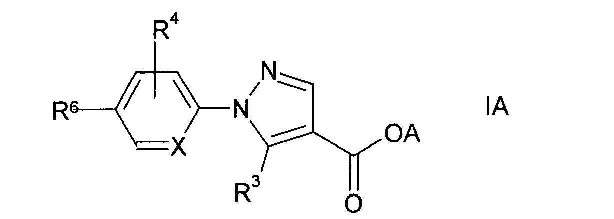 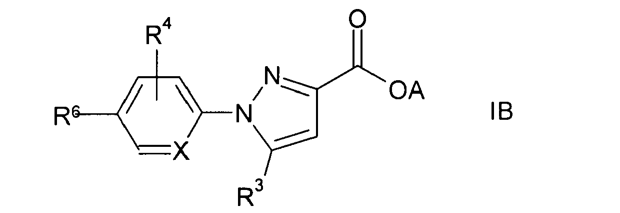 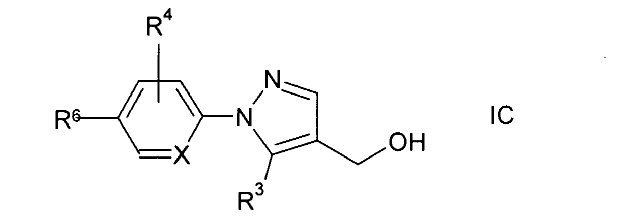 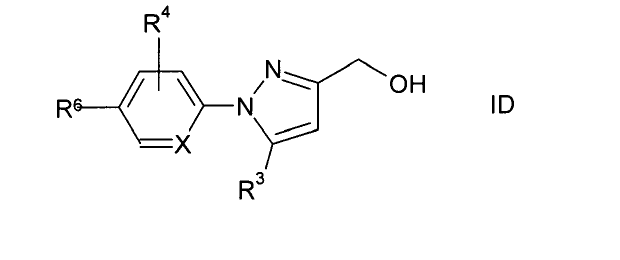 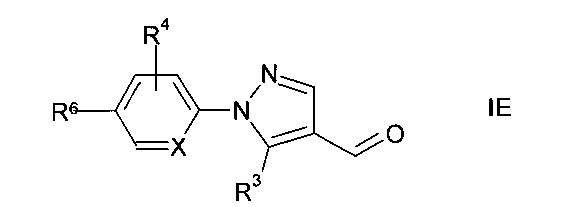 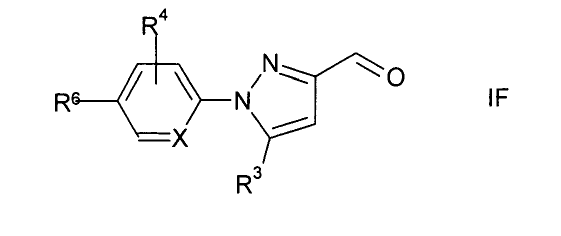 in which
R³, R⁴, R⁶ and X have the meanings indicated in Claim 1.

8. Process for the preparation of compounds of the formula IA 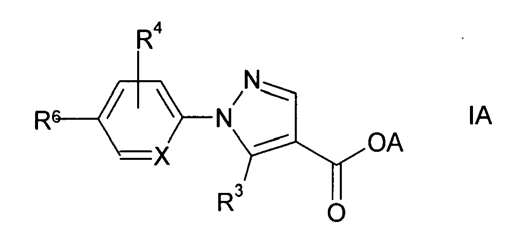 in which R³, R⁴, R⁶, X and A have the meaning indicated in Claim 1 and their salts and solvates, which is **characterised in that** a compound of the formula II 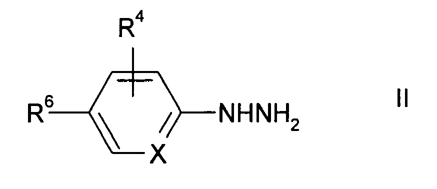 or its acid-addition salts
in which
R⁴, R⁶ and X have the meanings indicated in Claim 1,
is reacted with a compound of the formula III 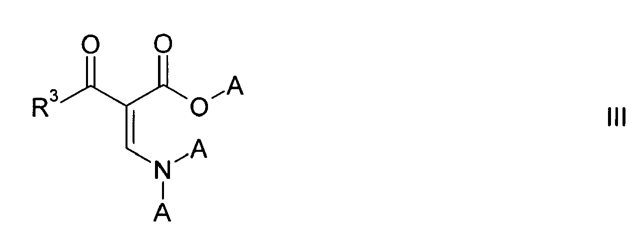 in which
A and R³ have the meanings indicated in Claim 1,
and/or **in that** a basic compound of the formula IA is converted into one of its salts by treatment with an acid.

9. Process for the preparation of compounds of the formula IB 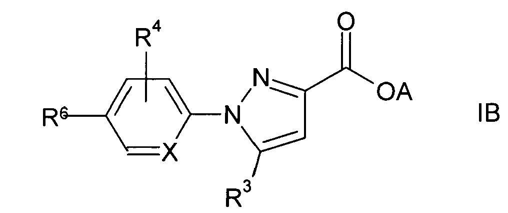 in which R³, R⁴, R⁶, X and A have the meaning indicated in Claim 1 and their salts and solvates, which is **characterised in that** a compound of the formula II 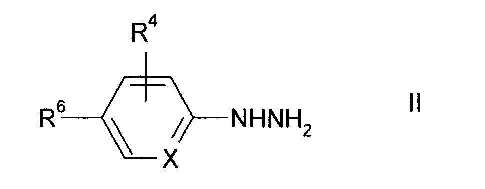 or its acid-addition salts
in which
R⁴, R⁶ and X have the meanings indicated in Claim 1,
is reacted with a compound of the formula IV 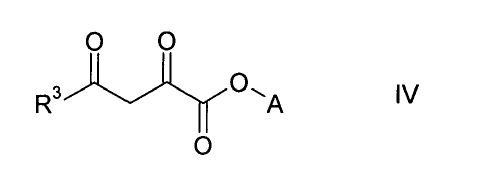 in which
A and R³ have the meanings indicated in Claim 1,
and/or **in that** a basic compound of the formula IB is converted into one of its salts by treatment with an acid.

10. Compounds of the formula I according to one or more of Claims 1 to 6 and their physiologically acceptable salts and solvates as medicaments.

11. Use of the compounds of the formula I according to one or more of Claims 1 to 6, and their salts and solvates, for the preparation of a medicament for the treatment and prophylaxis of diseases which can be influenced by the binding of the compounds of the formula I to 5 HT receptors.

12. Use of compounds of the formula I according to one or more of Claims 1 to 6 and/or their physiologically acceptable salts and solvates for the preparation of a medicament having a 5-HT receptor-antagonistic action.

13. Use of compounds of the formula I according to one or more of Claims 1 to 6 and/or their physiologically acceptable salts and solvates for the preparation of a medicament having a 5-HT2A receptor-antagonistic action.

14. Pharmaceutical composition, **characterised by** a content of at least one compound of the formula I according to one or more of Claims 1 to 6 and/or one of its physiologically acceptable salts and/or one of its solvates.

15. Process for the preparation of pharmaceutical compositions, **characterised in that** a compound of the formula I according to Claim 1 and/or one of its physiologically acceptable salts and/or one of its solvates is brought into a suitable dosage form together with at least one solid, liquid or semi-liquid excipient or adjuvant.

16. Use of compounds of the formula I according to one or more of Claims 1 to 6 and/or their physiologically acceptable salts or solvates for the preparation of a medicament for the prophylaxis and/or treatment of psychoses, neurological disorders, amyotrophic lateral sclerosis, eating disorders, such as bulimia, anorexia nervosa, of premenstrual syndrome and/or for positively influencing obsessive-compulsive disorder (OCD).

17. Compounds of the formula I in which R¹, R², R³, R⁴, R⁶ and X have the meaning indicated in Claim 1 and Het in the radicals R¹, R³ and R⁶ denotes one of the following radicals:
| | | | | | |
|---|---|---|---|---|---|
| 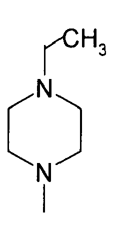 | 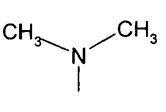 | 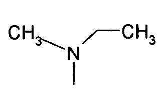 | 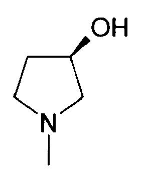 | 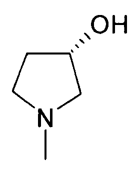 | 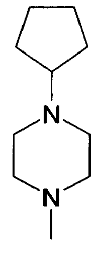 |
| 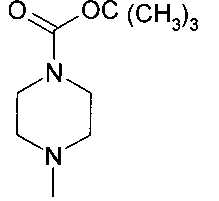 | | 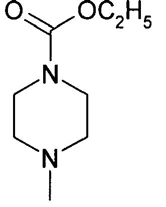 | | 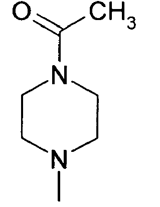 | |

## Revendications

1. Composés de formule I 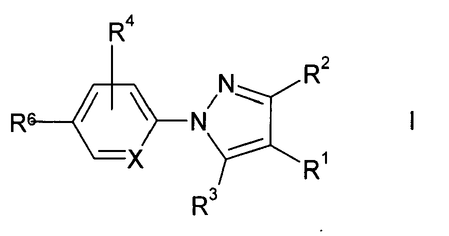 dans laquelle
R², R⁴ désignent H, A, Hal, cycloalkyle ayant 3 à 7 atomes de C, CF₃, NO₂, CN, OCF₃, OA, NHA, NA₂, NH₂,
R³, R⁶ désignent (CH₂)ₙHét, (CH₂)ₙAr,
R¹ désigne H ou un radical organique, en particulier (CH₂)ₙCO₂R⁵, (CH₂)ₙCOHét, CHO, (CH₂)ₙOR⁵, (CH₂)ₙHét, (CH₂)ₙN(R⁵)₂, CH=N-OA, CH₂CH=N-OA, (CH₂)ₙNHOA, (CH₂)ₙ(R⁵)Hét, (CH₂)ₙCH=N-Hét, (CH₂)ₙOCOR⁵, (CH₂)ₙN(R⁵)CH₂CH₂OR⁵, (CH₂)ₙN(R⁵)CH₂CH₂OCF₃, (CH₂)ₙN(R⁵)C(R⁵)COOR⁵, (CH₂)ₙN(R⁵)CH₂COHét, (CH₂)ₙN(R⁵)CH₂Hét, (CH₂)ₙN(R⁵)CH₂CH₂Hét, (CH₂)ₙN(R⁵)CH₂CH₂N(R⁵)CH₂COOR⁵, (CH₂)ₙN(R⁵)CH₂CH₂N(R⁵)₂, CH=CHCOOR⁵, CH=CHCH₂NR⁵Hét, CH=CHCH₂N(R⁵)₂, CH=CHCH₂OR⁵ ou (CH₂)ₙN(R⁵)Ar,
R⁵ désigne H ou A
A désigne alkyle ou alcoxy ayant 1 à 10 atomes de C, alcényle ou alcényloxyalkyle ayant 2 à 10 atomes de C, à chaîne linéaire ou ramifiée,
Hét désigne un radical organique saturé, insaturé ou aromatique mono- ou bicyclique hétérocyclique contenant un ou plusieurs hétéroatomes qui est non substitué ou mono- ou polysubstitué par A et/ou Hal,
Ar désigne un radical phényle qui est non substitué ou mono- ou polysubstitué par A et/ou Hal, OR⁵, OOCR⁵, COOR⁵, CON(R⁵)₂, CN, NO₂, NH₂, NHCOR⁵, CF₃ ou SO₂CH₃,
n vaut 0, 1, 2, 3, 4 ou 5
et
Hal désigne F, CI, Br ou 1
et
X désigne CH ou N, où R² et R⁴ ne désignent pas simultanément H dans le cas où X a la signification CH,
et leurs sels et solvats, énantiomères, racémates et autres mélanges d'énantiomères, en particulier leurs sels physiologiquement tolérés et solvats.

2. Composés de formule I selon la revendication 1, dans laquelle R⁶ désigne phényle, 2-, 3- ou 4-cyanophényle, 2-, 3- ou 4-fluorophényle, 2-, 3- ou 4-méthyl-, -éthyl-,-n-propyl- ou -n-butylphényle, 2,3-, 2,4-, 2,5-, 2,6-, 3,4-, 3,5- ou 3,6-difluoro-, -dichloro- ou -dicyanophényle, 3,4,5-trifluorophényle, 3,4,5-triméthoxy- ou -triéthoxyphényle, thiophén-2-yle ou thiophén-3-yle.

3. Composés de formule 1 selon l'une ou plusieurs parmi les revendications précédentes, dans laquelle R⁴ désigne H, Hal, CN, A ou NO₂.

4. Composés de formule 1 selon l'une ou plusieurs parmi les revendications précédentes, dans laquelle R² désigne H ou alkyle.

5. Composés de formule 1 selon l'une ou plusieurs parmi les revendications précédentes, dans laquelle R³ désigne phényle, 2-, 3- ou 4-cyanophényle, 2-, 3- ou 4-fluorophényle, 2-, 3- ou 4-méthyl-, -éthyl-, -n-propyl- ou -n-butylphényle, 2,3-, 2,4-, 2,5-, 2,6-difluoro- ou -dicyanophényle, thiophén-2-yle ou thiophén-3-yle, 2-, 3- ou 4-pyridyle, 2-, 4- ou 5-oxazolyle, 2-, 4- ou 5-thiazolyle, quinoléinyle, isoquinoléinyle, 2- ou 4-pyridazyle, 2-, 4- ou 5-pyrimidyle, 2- ou 3-pyrazinyle ou 2- ou 3-furanyle.

6. Composés de formule I selon l'une ou plusieurs parmi les revendications précédentes, dans laquelle X a la signification N.

7. Composés de formules lA, IB, IC, ID, lE et IF: 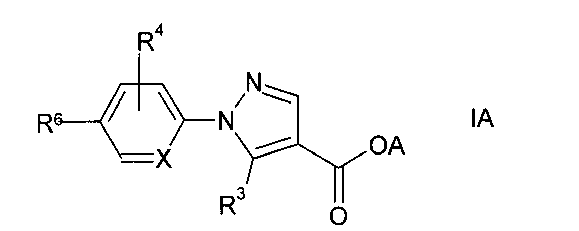 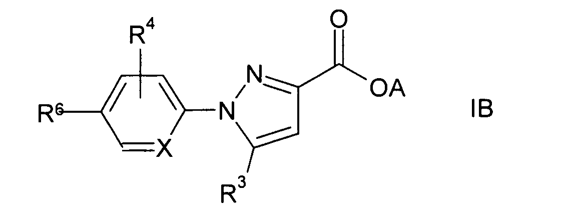 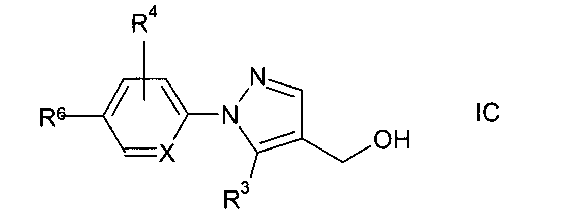 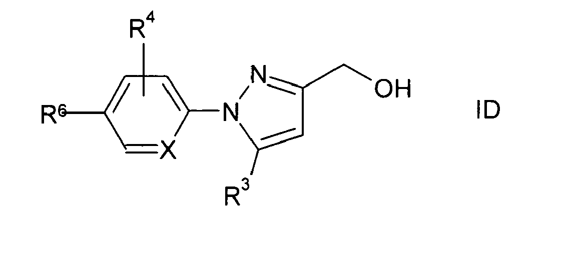 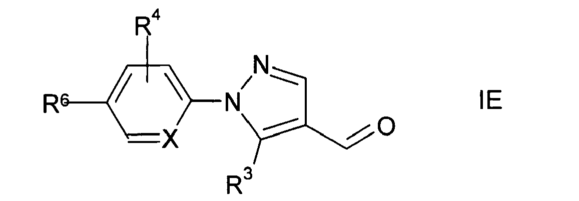 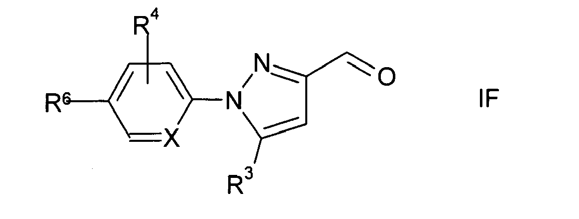 dans laquelle
R³, R⁴, R⁶ et X ont les significations indiquées selon la revendication 1.

8. Procédé de préparation de composés de formule lA 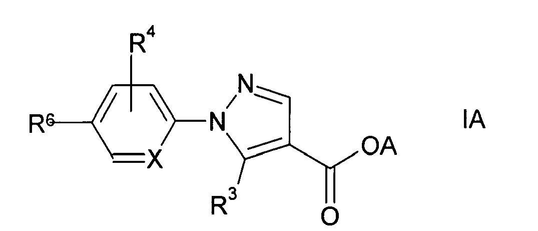 dans laquelle R³, R⁴, R⁶, X et A ont la signification indiquée selon la revendication 1
et leurs sels et solvats, qui est **caractérisé en ce qu'**un composé de formule Il 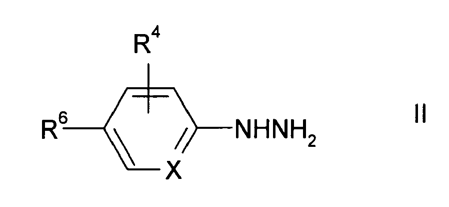 ou ses sels d'addition d'acide
dans laquelle
R⁴, R⁶ et X ont les significations indiquées selon la revendication 1, est réagi avec un composé de formule III 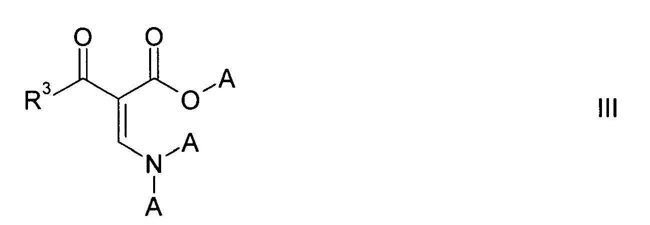 dans laquelle
A et R³ ont les significations indiquées selon la revendication 1,
et/ou **en ce qu'**un composé basique de formule lA est converti en l'un de ses sels par un traitement par un acide.

9. Procédé de préparation de composés de formule IB 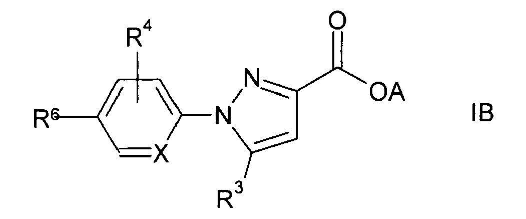 dans laquelle R³, R⁴, R⁶, X et A ont la signification indiquée selon la revendication 1
et leurs sels et solvats, qui est **caractérisé en ce qu'**un composé de formule II 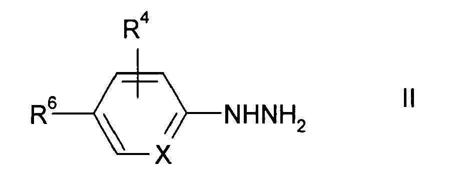 ou ses sels d'addition d'acide
dans laquelle
R⁴, R⁶ et X ont les significations indiquées selon la revendication 1, est réagi avec un composé de formule IV 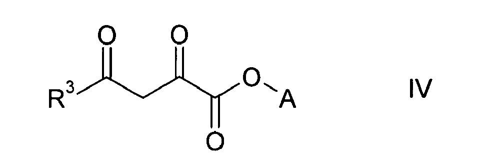 dans laquelle
A et R³ ont les significations indiquées selon la revendication 1,
et/ou **en ce qu'**un composé basique de formule IB est converti en l'un de ses sels par un traitement par un acide.

10. Composés de formule 1 selon l'une ou plusieurs parmi les revendications 1 à 6 et leurs sels physiologiquement acceptables et solvats comme médicaments.

11. Utilisation des composés de formule 1 selon l'une ou plusieurs parmi les revendications 1 à 6, et de leurs sels et solvats, pour la préparation d'un médicament destiné au traitement et à la prophylaxie de maladies pouvant être influencées par la fixation des composés de formule 1 aux récepteurs 5HT.

12. Utilisation de composés de formule 1 selon l'une ou plusieurs parmi les revendications 1 à 6 et/ou de leurs sels physiologiquement acceptables et solvats pour la préparation d'un médicament ayant une action antagoniste du récepteur 5HT.

13. Utilisation de composés de formule I selon l'une ou plusieurs parmi les revendications 1 à 6 et/ou de leurs sels physiologiquement acceptables et solvats pour la préparation d'un médicament ayant une action antagoniste du récepteur 5HT2A.

14. Composition pharmaceutique, **caractérisée par** une teneur en un au moins un composé de formule 1 selon l'une ou plusieurs parmi les revendications 1 à 6 et/ou l'un de ses sels physiologiquement acceptables et/ou l'un de ses solvats.

15. Procédé de préparation de compositions pharmaceutiques, **caractérisé en ce qu'**un composé de formule 1 selon la revendication 1 et/ou l'un de ses sels physiologiquement acceptables et/ou l'un de ses solvats est amené sous une forme de dosage convenable, conjointement avec au moins un excipient ou adjuvant solide, liquide ou semi-liquide.

16. Utilisation de composés de formule I selon l'une ou plusieurs parmi les revendications 1 à 6 et/ou de leurs sels physiologiquement acceptables ou solvats pour la préparation d'un médicament destiné à la prophylaxie et/ou au traitement de psychoses, de troubles neurologiques, de la sclérose latérale amyotrophique, de troubles de l'alimentation, tels que la boulimie, l'anorexie mentale, du syndrome prémenstruel et/ou pour influencer de manière positive le trouble obsessionnel-compulsif (TOC).

17. Composés de formule I dans laquelle R¹, R², R³, R⁴, R⁶ et X ont la signification indiquée selon la revendication 1 et Hét dans les radicaux R¹, R³ et R⁶ désigne l'un des radicaux suivants :
| | | | | | |
|---|---|---|---|---|---|
| 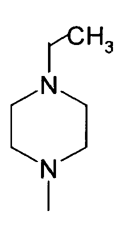 | 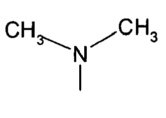 | 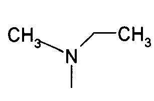 | 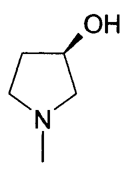 | 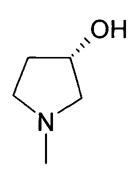 | 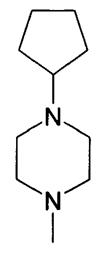 |
| 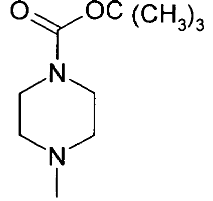 | | 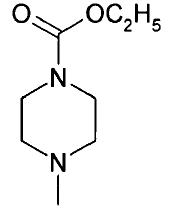 | | 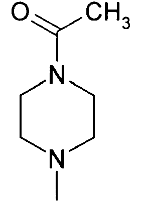 | |
